(19) Europäisches Patentamt
European Patent Office
Office européen des brevets

(11) EP 3 197 563 B1

(12) **EUROPEAN PATENT SPECIFICATION**

(45) Date of publication and mention of the grant of the patent:
**30.10.2019 Bulletin 2019/44**

(21) Application number: **15750418.4**

(22) Date of filing: **12.08.2015**

(51) Int Cl.:
***A61K 8/92*** (2006.01) ***A61K 8/02*** (2006.01)
***A61K 8/26*** (2006.01) ***A61Q 17/04*** (2006.01)

(86) International application number:
**PCT/EP2015/068567**

(87) International publication number:
**WO 2016/030193 (03.03.2016 Gazette 2016/09)**

(54) **COMPOSITION BASED ON A LIPOPHILIC ORGANIC UV-SCREENING AGENT AND A FILLER**

ZUSAMMENSETZUNG AUF DER BASIS EINES LIPOPHILEN ORGANISCHEN SONNENSCHUTZMITTELS UND EINES FÜLLSTOFFES

COMPOSITION BASÉE SUR UN FILTRE UV ORGANIQUE LIPOPHILE ET UNE CHARGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2014 FR 1458015**

(43) Date of publication of application:
**02.08.2017 Bulletin 2017/31**

(73) Proprietor: **L'OREAL**
**75008 Paris (FR)**

(72) Inventors:
- **BAILLEUL, Michael**
**94152 CHEVILLY LA RUE (FR)**
- **LE GARS, Gwénola**
**F-75013 Paris (FR)**
- **MAC DERMOTT, Padraig**
**F-92190 Meudon (FR)**

(74) Representative: **L'Oreal**
**Service D.I.P.I.**
**9, rue Pierre Dreyfus**
**92110 Clichy (FR)**

(56) References cited:
**WO-A1-2011/067807 WO-A1-2013/190709**
**FR-A1- 2 982 148 US-A1- 2006 013 787**
**US-A1- 2007 048 239**

- **Anonymous: "AGRANA STARCH Compact Lightening Powder "Caramel Shine" with AGENASORB DRY RICEGEL NS 9095 "Formulated and developed exclusively by Eckart GmbH" Ingredients INCI-Name %w/w Supplier", , 5 January 2013 (2013-01-05), XP055184352, Retrieved from the Internet: URL:www.agrana.com [retrieved on 2015-04-20]**
- **Anonymous: "Belides", , 11 June 2012 (2012-06-11), pages 1-16, XP055184344, Retrieved from the Internet: URL:http://web.archive.org/web/20120611014049/http://www.drskinspa.com/Ageless Derma/Belides-Daisy-Flower.pdf [retrieved on 2015-04-20]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 197 563 B1

## Description

The present invention relates to a composition, in particular anti-sun composition, which is preferably solid, in particular in the form of a loose or compact powder, comprising at least one pulverulent phase and at least one liquid fatty phase comprising at least one lipophilic organic UV-screening agent, the pulverulent phase comprising at least one particular filler. The present invention also relates to a process for coating keratin materials.

It is known that radiation with wavelengths of between 280 nm and 400 nm permits tanning of the human epidermis and that radiation with wavelengths between 280 and 320 nm, known as UVB rays, harm the development of a natural tan. Such exposure is also liable to induce impairment in the biomechanical properties of the epidermis, which is reflected by the appearance of wrinkles, leading to premature aging of the skin.

It is also known that UVA rays with wavelengths of between 320 and 400 nm penetrate more deeply into the skin than UVB rays. UVA rays bring about immediate and persistent tanning of the skin. Daily exposure to UVA rays, even of short duration, under normal conditions can result in damage to the collagen fibers and the elastin, which is reflected by a modification to the microrelief of the skin, the appearance of wrinkles and uneven pigmentation (liver spots, heterogeneity of the complexion).

Many cosmetic compositions for photoprotecting the skin (against UVA and/or UVB) have been proposed to date. Formulations that are easy for the users to apply to the skin are most particularly sought. These **UV**-screening cosmetic compositions must moreover satisfy the regulations in terms of protection factor and in particular the European regulation on anti-sun products, in particular on the protection ratio between UVB and UVA and more particularly the SPF/PPD ratio, which must be less than 3.

The efficacy of anti-sun compositions for UVB protection is generally expressed by the sun protection factor (SPF), which is expressed mathematically by the ratio of the dose of UV radiation necessary to reach the erythema-forming threshold with the UV-screening agent to the dose of UV radiation necessary to reach the erythema-forming threshold without UV-screening agent. This factor thus concerns the efficacy of the protection, the biological spectrum of action of which is centered in the UVB range, and consequently accounts for the protection with respect to this UVB radiation.

To characterize the protection with respect to UVA, the PPD (persistent pigment darkening) method, which measures the skin color observed 2 to 4 hours after exposure of the skin to UVA radiation, is particularly recommended and used. This method has been adopted since 1996 by the Japanese Cosmetic Industry Association (JCIA) as official test procedure for the UVA labeling of products and is frequently used by test laboratories in Europe and the United States; (Japan Cosmetic Industry Association Technical Bulletin. Measurement Standards for UVA protection efficacy. Issued November 21, 1995 and effective as of January 1, 1996).

The UVAPPD sun protection factor (UVAPPD PF) is expressed mathematically by the ratio of the UVA radiation dose necessary to reach the pigmentation threshold with the UV-screening agent (MPPDp) to the UVA radiation dose necessary to reach the pigmentation threshold without UV-screening agent (MPPDnp).

$$\text{FP UVA}_{\text{PPD}} = \frac{\text{MPPDp}}{\text{MPPDnp}}$$

Anti-sun compositions are conventionally in the form of an emulsion of oil-in-water type (i.e. a cosmetically acceptable support consisting of an aqueous dispersing continuous phase and of an oily dispersed discontinuous phase) or of the water-in-oil type (i.e. a cosmetically acceptable support consisting of an oily dispersing continuous phase and of an aqueous dispersed discontinuous phase) which contains, in various concentrations, one or more standard lipophilic and/or hydrophilic organic UV-screening agents, which are capable of selectively absorbing harmful UV rays, these UV-screening agents (and the amounts thereof) being selected as a function of the desired sun protection factor.

However, the incorporation of organic UV-screening agents into this type of cosmetic composition occasionally leads to an uncomfortable cosmetic feel, in particular a tacky effect during application to the skin, and which persists over time. These cosmetic compositions containing organic screening agents have a tendency to leave a shiny film on the surface of the skin.

The shiny effect provided by lipophilic organic UV-screening agents is all the greater, the higher their content in the compositions. It is therefore particularly important for anti-sun compositions to have high levels of SPF and PPD protection.

In the field of anti-sun cosmetics, the galenical form in the powder form makes it possible to avoid all these disadvantages due to the presence of fillers which make it possible to introduce softness and mattness. Loose or compact photoprotective powders based on inorganic UV screening agents which are metal oxide pigments, such as titanium dioxide or zinc oxide, are in particular known. The introduction of these inorganic screening agents into these galenical forms results in significant coverage and in a loss of the transparency of the product which leaves a whitening film on

the skin. There also exist, in Patent Application EP 0 839 518, cosmetic compositions in the loose or compact powder form comprising organic **UV**-screening agents entrapped in a porous spherical silica aggregate. This type of product is less covering than the preceding one. Nevertheless, the content of screening agents which can be introduced into this type of galenical form remains low. For this reason, the efficacy (SPF) of this type of product remains limited.

However, galenical forms in powder form do not produce a satisfactory efficacy (SPF). In particular, inorganic UV-screening agents did not make it possible to achieve high sun protections (high SPFs), hence the need to use a combination of inorganic UV-screening agents and lipophilic organic UV-screening agents. Furthermore, increasing the content of organic lipophilic screening agent(s) results in a problem of cosmeticity of the composition in powder form, in particular due to waxing of the powder preventing any disintegration.

Patent application WO 2013/068236 describes cosmetic compositions in powder form containing lipophilic organic screening agents. The amount of screening agent that can be introduced is high (>7% by weight relative to the total weight of the composition) and the efficacy (SPF) is then high. However, the introduction of lipophilic screening agents has a tendency to degrade the cosmeticity of the powders, and creates in particular problems in terms of taking up the product, waxing, tackiness and non-homogeneity.

There remains therefore a need to carry out improvements to anti-sun cosmetic compositions in powder form with good cosmetic, spreading and softness properties, without waxing and which are homogeneous, while at the same time comprising a high concentration of sunscreen.

The applicant has discovered, surprisingly, that at least one of these objectives can be achieved by using fillers having an oil absorption capacity, measured at the wet point, of at least 70 ml/100 g and a size expressed in volume-average diameter of at least 15 microns.

For the purposes of the present invention, the absorption capacity measured at the wet point, denoted Wp, corresponds to the amount of oil which it is necessary to add to 100 g of particles in order to obtain a homogeneous paste. It is measured according to the "wet point" method or the method for determining the oil uptake of a powder described in standard ISO 787-5:1980. It corresponds to the amount of oil adsorbed onto the available surface of the powder and/or absorbed by the powder by measurement of the wet point, described below:
An amount m = 2 g of powder is placed on a glass plate, and the oil (isononyl isononanoate) is then added dropwise. After addition of 4 to 5 drops of oil to the powder, mixing is carried out using a spatula, and addition of oil is continued until conglomerates of oil and powder have formed. From this point, the oil is added at the rate of one drop at a time and the mixture is subsequently triturated with the spatula. The addition of oil is stopped when a firm, smooth paste is obtained. This paste must be able to be spread on the glass plate without cracking or forming lumps. The volume Vs (expressed in ml) of oil used is then noted. The oil uptake corresponds to the ratio Vs/m.

The sizes of the filler particles according to the invention may be measured by static light scattering using a commercial particle size analyzer such as the MasterSizer 2000 machine from Malvern. The data are processed on the basis of the Mie scattering theory.

This theory, which is exact for isotropic particles, makes it possible to determine, in the case of non-spherical particles, an "effective" particle diameter. This theory is in particular described in the publication by Van de Hulst, H.C., Light Scattering by Small Particles, Chapters 9 and 10, Wiley, New York, 1957.

The present invention thus relates to a composition, preferably a solid composition, in particular in the form of a loose or compact powder, comprising:

a) at least one pulverulent phase present in a content greater than or equal to 50% by weight relative to the total weight of the composition,
b) at least one liquid fatty phase comprising at least one lipophilic organic UV-screening agent, in which the pulverulent phase comprises at least one filler having an oil absorption capacity, measured at the wet point, of greater than or equal to 70 ml/100 g and a size expressed as volume-average diameter of greater than or equal to 15 microns; the said filler being chosen from the family of micas, in particular natural or synthetic micas and
c) optionally at least one additional filler having either an oil absorption capacity, measured at the wet point, strictly less than 70 ml/100 g, or a size expressed as volume-average diameter strictly less than 15 microns, or both ;

the sizes expressed as volume-average diameter of filler particles being measured by static light scattering using a commercial particle size analyzer.

A composition in accordance with the invention is formulated in a cosmetically acceptable medium.

For the purposes of the present invention:

the term "cosmetically acceptable medium" is intended to mean any medium that is compatible with the skin and/or its appendages, which has a pleasant color, odor and feel, and which does not cause any unacceptable discomfort (stinging, tautness or redness) liable to discourage the consumer from using this composition;

the term "solid" is intended to mean the state of the composition at ambient temperature (25°C) and at atmospheric pressure (1 atm), i.e. a composition of high consistency, which retains its form during storage. As opposed to "fluid" compositions, it does not flow under its own weight;

the term "compact powder" is intended to mean a mass of product of which the cohesion is at least partly provided by compacting during the manufacture. In particular, it should more specifically be understood that these powders have a Shore A hardness, measured using a Zwick durometer, which ranges, depending on the strength of the tints under consideration, from 12 to 50° Shore A, preferably from 15 to 25° Shore A;

the term "loose powder" is intended to mean a mass of product which is capable of collapsing under its own weight, such a mass being formed of particles which are predominantly isolated and movable with respect to one another;

the term "liquid" is intended to mean liquid at ambient temperature (25°C) and atmospheric pressure (1 atm);

the term "lipophilic organic UV-screening agent" is intended to mean an organic molecule that is capable of screening out UV radiation between 290 and 400 nm, and which can be dissolved in molecular form or dispersed in an oily phase in order to obtain a macroscopically homogeneous phase;

the term "insoluble UV-screening agent" is intended to mean any UV-screening agent that may be in the form of particles in a liquid fatty phase and in a liquid aqueous phase, such as insoluble organic UV-screening agents and inorganic UV-screening agents;

the term "human keratin materials", is intended to mean the skin (body, face, area around the eyes), hair, eyebrows, eyelashes, body hair, nails, lips, mucous membranes, preferably the skin (body, face, area around the eyes);

the term "filler" is intended to mean colorless or white, mineral or synthetic particles of any shape, which are insoluble or dispersed in the medium of the composition irrespective of the temperature at which the composition is manufactured. They are mineral or organic in nature and make it possible to confer on the composition softness, mattness and uniformity on the skin;

the term "spherical" is intended to mean having the shape or substantially the shape of a sphere, i.e. having a sphericity index, i.e. the ratio between the largest diameter and the smallest diameter, of less than 1.2;

the term "lamellar" is intended to mean of parallelepipedal shape (rectangular or square surface), discoid shape (circular surface) or ellipsoid shape (oval surface), and characterized by three dimensions: a length, a width and a height;

the term "pigments" is intended to mean white or colored, mineral or organic particles of any shape, which are insoluble in physiological medium, and which are intended to color the composition;

the term "nacres" is intended to mean colored particles of any shape, which may or may not be iridescent, in particular produced by certain molluscs in their shell, or alternatively synthesized, and which have a color effect via optical interference;

the term "liposoluble dyes" is intended to mean compounds which are generally organic and which are soluble in fatty substances such as oils and capable of coloring;

the term "water-soluble dye" is intended to mean any natural or synthetic, generally organic compound, which is soluble in an aqueous phase or in water-miscible solvents and which is capable of coloring;

the term "volatile oil" is intended to mean an oil that is capable of evaporating on contact with the skin or the keratin fiber in less than one hour, at ambient temperature and atmospheric pressure. The volatile oils of the invention are volatile cosmetic oils that are liquid at ambient temperature, with a non-zero vapor pressure, at ambient temperature and atmospheric pressure, ranging in particular from 0.13 Pa to 40 000 Pa, in particular ranging from 1.3 Pa to 13 000 Pa and more particularly ranging from 1.3 Pa to 1300 Pa;

the term "nonvolatile oil" is intended to mean an oil that remains on the skin or the keratin fiber at ambient temperature and atmospheric pressure for at least several hours, and that in particular has a vapor pressure of less than 0.13 Pa.

According to preferred embodiments, subjects of the present invention are also the following technical characteristics which correspond to at least one of the technical problems mentioned, considered alone or in combination:

- the filler(s) having an oil absorption capacity, measured at the wet point, of at least 70 ml/100 g and a size expressed as volume-average diameter of at least 15 microns is (are) present according to a total content of at least 5% by weight, relative to the total weight of the composition, preferably ranging from 10% to 50% by weight, in particular from 20% to 40% by weight, and better still from 25% 35% by weight, relative to the total weight of the composition;
- the filler(s) having an oil absorption capacity, measured at the wet point, of at least 70 ml/100 g and a size expressed as volume-average diameter of at least 15 microns is (are) present according to a total content of at least 5% by weight, relative to the total weight of the pulverulent phase, preferably ranging from 15% to 60% by weight, in particular from 25% to 50% by weight, and better still from 30% to 45% by weight, relative to the total weight of the pulverulent phase;
- the filler(s) having an oil absorption capacity, measured at the wet point, of at least 70 ml/100 g and a size expressed as volume-average diameter of at least 15 microns is (are) chosen from the family of micas, in particular natural or synthetic micas;
- the pulverulent phase comprises at least one additional filler;
- the additional filler(s) is (are) present according to a total content of at least 5% by weight, relative to the total weight of the composition, preferably ranging from 10% to 50% by weight, in particular from 20% to 40% by weight, relative to the total weight of the composition;
- the additional filler(s) is (are) present according to a total content ranging from 15% to 60% by weight, relative to the total weight of the pulverulent phase, preferably ranging from 25% to 50% by weight, in particular from 30% to 45% by weight, relative to the total weight of the pulverulent phase;
- the additional filler(s) is (are) chosen from polyamide powders, organopolysiloxane elastomer powders, metal soaps, silicas, aerogel particles, metal (poly)oxides, talcs, micas, and mixtures thereof;
- the lipophilic organic **UV**-screening agent(s) is (are) present in a total content greater than or equal to 0.5% by weight, relative to the total weight of the composition, in particular ranging from 1% to 25% by weight, preferably ranging from 4% to 20% by weight, even better still from 7% to 20% by weight, relative to the total weight of the composition;
- the lipophilic organic **UV**-screening agent(s) is (are) present in a total content greater than or equal to 40% by weight, in particular ranging from 50% to 70% by weight, relative to the total weight of the liquid fatty phase;
- the lipophilic organic **UV**-screening agent(s) is (are) chosen from cinnamic derivatives; anthranilates; salicylic derivatives, dibenzoylmethane derivatives, camphor derivatives; benzophenone derivatives; $\beta,\beta$-diphenyl acrylate derivatives; triazine derivatives; benzotriazole derivatives; phenyl benzotriazole derivatives such as drometrizole trisiloxane; benzalmalonate derivatives; imidazolines; p-aminobenzoic acid derivatives; benzoxazole derivatives; screening polymers and screening silicones; $\alpha$-alkylstyrene-derived dimers; 4,4-diarylbutadienes; merocyanine derivatives and mixtures thereof, preferably comprising at least one cinnamic derivative, such as ethylhexyl methoxycinnamate, at least one triazine derivative, such as bis-ethylhexyloxyphenol methoxyphenyl triazine or ethylhexyl triazone;
- the lipophilic organic **UV**-screening agent(s) is (are) chosen from butyl methoxy dibenzoylmethane, ethylhexyl methoxycinnamate, ethylhexyl salicylate, homosalate, butyl methoxydibenzoylmethane, octocrylene, benzophenone-3, n-hexyl 2-(4-diethylamino-2-hydroxybenzoyl)benzoate, 4-methylbenzylidenecamphor, bis-ethylhexyloxyphenol methoxyphenyl triazine, ethylhexyl triazone, diethylhexyl butamido triazone, 2,4,6-tris(dineopentyl 4'-amino benzalmalonate)-s-triazine, 2,4,6-tris(diisobutyl 4'-aminobenzalmalonate)-s-triazine, 2,4-bis(dineopentyl 4'-aminobenzalmalonate)-6-(n-butyl 4'-aminobenzoate)-s-triazine, 2,4,6-tris(biphenyl-4-yl)-1,3,5-triazine, 2,4,6-tris(terphenyl)-1,3,5-triazine, drometrizole trisiloxane, polysilicone-15, 1,1-dicarboxy(2,2'-dimethylpropyl)-4,4-diphenylbutadiene, 2,4-bis[5-1(dimethylpropyl)benzoxazol-2-yl(4-phenyl)imino]-6-(2-ethylhexyl)imino-1,3,5-triazine, and mixtures thereof;
- the liquid fatty phase may comprise at least one oil, preferably nonvolatile oil, in particular chosen from hydrocarbon-based and silicone oils, and mixtures thereof;
- the total content of oil(s) is greater than or equal to 1% by weight, in particular ranges from 1% to 15% by weight, relative to the total weight of the composition;
- the total content of oil(s) is greater than or equal to 5% by weight, in particular ranges from 10% to 50% by weight, relative to the total weight of the liquid fatty phase;
- the pulverulent phase comprises at least one insoluble UV-screening agent, in particular chosen from metal (poly)oxides, in particular titanium (poly)oxides;
- the insoluble UV-screening agent(s) is (are) present in a total content greater than or equal to 2% by weight, relative to the total weight of the composition, in particular ranging from 5% to 30% by weight, preferably from 5% to 10% by weight, relative to the total weight of the composition;

- the insoluble UV-screening agent(s) is (are) present in a total content greater than or equal to 2% by weight, relative to the total weight of the pulverulent phase, in particular ranging from 5% to 40% by weight, preferably from 5% to 15% by weight, relative to the total weight of the pulverulent phase;
- the lipophilic organic UV-screening agent(s) and the inorganic UV-screening agent(s) are present in a respective weight content such that the ratio of the total content of lipophilic organic UV-screening agent(s) to the content of insoluble UV-screening agent(s) ranges from 0 to 10, preferably from 1 to 6;
- the pulverulent phase comprises at least one colorant, in particular chosen from pigments, nacres and dyes, and mixtures thereof;
- the pulverulent phase comprises at least one insoluble UV-screening agent, in particular chosen from metal (poly)oxides, in particular titanium (poly)oxides, and at least one colorant chosen from pigments, in particular from metal (poly)oxides, in particular titanium (poly)oxides, iron (poly)oxides, and mixtures thereof;
- the pulverulent phase comprises at least one additional filler, in particular a spherical filler, which is organic or mineral, preferably mineral, in particular chosen from metal (poly)oxides, preferably bismuth (poly)oxides, and at least one colorant chosen from nacres, such as nacreous pigments, in particular those based on bismuth oxychloride or coated with bismuth oxychloride.

According to another aspect, the present invention relates to a cosmetic assembly comprising a container delimiting one or more compartments and a composition as previously described, placed inside said compartment(s).

A composition according to the invention is preferably solid, in particular in the form of a loose or compacted powder, more particularly in compacted form. This composition comprises a pulverulent phase and a liquid fatty phase.

## PULVERULENT PHASE:

The composition according to the invention comprises at least one pulverulent phase. According to one embodiment of the invention, the composition comprises a pulverulent phase in a content greater than or equal to 50% by weight, in particular greater than or equal to 55% by weight, preferably ranging from 60% to 95% by weight and better still from 80% to 85% by weight, relative to the total weight of the composition.

The pulverulent phase comprises at least one filler having an oil absorption capacity, measured at the wet point, of at least 70 ml/100 g according to the method previously defined, and a size expressed as volume-average diameter of at least 15 microns according to the method previously defined.

The pulverulent phase may also comprise additional fillers, coloring agents, and insoluble UV-screening agents.

### - filler(s) according to the invention

The pulverulent phase comprises at least one filler according to the invention having an oil absorption capacity, measured at the wet point, of at least 70 ml/100 g according to the method previously defined, and a size expressed as volume-average diameter of at least 15 microns according to the method previously defined; the said filler being chosen from the family of micas, in particular natural or synthetic micas.

Surprisingly, the filler(s) according to the invention make(s) it possible to retain good cosmetic properties of the powder according to the invention, even at high concentrations of lipophilic organic screening agent(s).

The filler(s) according to the invention has (have) a size expressed as volume-average diameter of at least 15 microns. According to one preferred embodiment, the filler(s) has (have) a size expressed as volume-average diameter ranging from 15 to 150 microns, preferably ranging from 15 to 60 microns, and better still from 15 to 40 microns.

According to one embodiment, the filler(s) having an oil absorption capacity, measured at the wet point, of at least 70 ml/100 g and a size expressed as volume-average diameter of at least 15 microns is (are) of lamellar shape.

As filler in the family of micas having an oil absorption capacity, measured at the wet point, of at least 70 ml/100 g and a size expressed as volume-average diameter of at least 15 microns, mention may be made of Sumicos Velvet Mica 43037 sold by Sudarshan Chemical (INCI name: mica); Mearlmica DD sold by BASF Personal Care Ingredients (INCI name: mica); Mearlmica CF sold by BASF Personal Care Ingredients (INCI name: mica); and Synafil S 1050 sold by Eckart (INCI name: synthetic fluorphlogopite).

According to one preferred embodiment, the filler having an oil absorption capacity, measured at the wet point, of at least 70 ml/100 g and a size expressed as volume-average diameter of at least 15 microns is Sumicos Velvet Mica 43037 sold by Sudarshan Chemical (INCI name: mica).

According to one preferred embodiment, the filler having an oil absorption capacity, measured at the wet point, of at least 70 ml/100 g and a size expressed as volume-average diameter of at least 15 microns is Mearlmica DD sold by BASF Personal Care Ingredients (INCI name: mica).

According to one embodiment, the filler(s) having an oil absorption capacity, measured at the wet point, of at least 70 ml/100 g and a size expressed as volume-average diameter of at least 15 microns is (are) present in the

composition according to the invention in a content of at least 5% by weight, preferably in a content ranging from 10% to 50% by weight, in particular in a content ranging from 20% to 40% by weight, and better still from 25% to 35% by weight, relative to the total weight of the composition.

According to one embodiment, the filler(s) having an oil absorption capacity, measured at the wet point, of at least 70 ml/100 g and a size expressed as volume-average diameter of at least 15 microns is (are) present in the composition according to the invention in a content ranging from at least 5% by weight, relative to the total weight of the pulverulent phase, preferably in a content ranging from 15% to 60% by weight, preferably in a content ranging from 25% to 50% by weight, and better still from 30% to 45% by weight, relative to the total weight of the pulverulent phase.

In one preferred embodiment of the invention, the filler(s) having an oil absorption capacity, measured at the wet point, of at least 70 ml/100 g and a size expressed as volume-average diameter ranging from 15 to 150 microns, preferably from 15 to 40 microns is (are) present in the composition according to the invention in a content of at least 5% by weight, preferably in a content ranging from 10% to 50% by weight, in particular in a content ranging from 20% to 40% by weight, and better still from 25% to 35% by weight, relative to the total weight of the composition.

In one preferred embodiment of the invention, the filler(s) having an oil absorption capacity, measured at the wet point, of at least 70 ml/100 g and a size expressed as volume-average diameter ranging from 15 to 150 microns, preferably from 15 to 40 microns is (are) present in the composition according to the invention in a content of at least 5% by weight, preferably in a content ranging from 15% to 60% by weight, in particular in a content ranging from 25% to 50% by weight, and better still from 30% to 45% by weight, relative to the total weight of the pulverulent phase.

- **additional fillers**

The pulverulent phase according to the invention may contain at least one additional filler.

The term "additional filler(s)" is intended to mean at least one filler having either an oil absorption capacity, measured at the wet point, strictly less than 70 ml/100 g, according to the method previously defined, or a size expressed as volume-average diameter strictly less than 15 microns, as defined above, or both.

The additional filler(s) is (are) present in a total content greater than or equal to 5% by weight, relative to the total weight of the composition, in particular from 10% to 50% by weight, better still from 20% to 40% by weight, relative to the total weight of the composition.

The additional filler(s) is (are) present in a total content greater than or equal to 10% by weight, relative to the total weight of the pulverulent phase, in particular from 15% to 60% by weight, better still from 25% to 50% by weight, even better still from 30% to 45% by weight, relative to the total weight of the pulverulent phase.

The additional filler(s) used in the composition according to the present invention may have a lamellar (or platelet) or spherical (or globular) shape, the shape of a fiber or any other intermediate shape between these defined shapes.

According to one embodiment of the invention, the composition comprises at least one additional filler chosen from spherical fillers and lamellar fillers, and mixtures thereof. According to one preferred mode of the invention, the pulverulent phase comprises, as additional fillers, a mixture of spherical fillers and lamellar fillers.

◦ Spherical fillers

The spherical fillers used according to the invention have the shape or substantially the shape of a sphere and can be hollow or solid. Advantageously, the spherical fillers of the invention have a particle size (number-average diameter) ranging from 0.1 $\mu$m to 250 $\mu$m, preferably ranging from 1 $\mu$m to 150 $\mu$m and more preferentially from 10 $\mu$m to 100 $\mu$m.

The spherical particles can be organic or mineral microspheres.

Mention may be made, as spherical organic fillers, for example, of polyamide powders and in particular Nylon® powders, such as Nylon-1 or Polyamide 12, sold under the Orgasol names by Arkema; polyethylene powders; polytetrafluoroethylene (Teflon*) powders; microspheres based on acrylic copolymers, such as those made of ethylene glycol dimethacrylate/lauryl methacrylate copolymer sold by Dow Corning under the Polytrap name; expanded powders, such as hollow microspheres and in particular the microspheres sold under the Expancel name by Kemanord Plast or under the name Micropearl F 80 ED by Matsumoto; silicone resin microbeads, such as those sold under the Tospearl name by Toshiba Silicone; powders of a preferably non-emulsifying organopolysiloxane elastomer, such as those sold under the KSP 100 name by the company Shin Etsu (INCI name: vinyl dimethicone/methicone silsesquioxane crosspolymer), under the KSP-300 name by the company Shin Etsu (INCI name: diphenyl dimethicone/vinyl diphenyl dimethicone/silsesquioxane crosspolymer); polymethyl methacrylate microspheres, sold under the name Microsphere M-100 by Matsumoto or under the name Covabead LH85 by Wackherr; ethylene/acrylate copolymer powders, such as those sold under the Flobeads name by Sumitomo Seika Chemicals; powders formed of natural organic materials, such as starch powders, in particular powders formed of maize, wheat or rice starch, which are or are not crosslinked, such as

powders formed of starch crosslinked with octenylsuccinic anhydride, sold under the Dry-Flo name by National Starch; metal soaps derived from organic carboxylic acids having from 8 to 22 carbon atoms, preferably from 12 to 18 carbon atoms, for example zinc stearate, magnesium stearate, lithium stearate, zinc laurate or magnesium myristate; Polypore L 200 (Chemdal Corporation); polyurethane powders, in particular powders formed of crosslinked polyurethane comprising a copolymer, said copolymer comprising trimethylol hexyllactone, such as the hexamethylene diisocyanate/trimethylol hexyllactone polymer sold under the name Plastic Powder D-400® or Plastic Powder D-800® by Toshiki; carnauba microwaxes, such as that sold under the name MicroCare 350® by Micro Powders; synthetic wax microwaxes, such as that sold under the name MicroEase 114S® by Micro Powders; microwaxes composed of a mixture of carnauba wax and polyethylene wax, such as those sold under the names Micro Care 300® and 310® by Micro Powders; microwaxes consisting of a mixture of carnauba wax and synthetic wax, such as that sold under the name Micro Care 325® by Micro Powders; or polyethylene microwaxes, such as those sold under the names Micropoly 200®, 220®, 220L® and 250S® by Micro Powders.

As mineral spherical filler, mention may in particular be made of aerogel particles.

The aerogel particle(s) used in the present invention advantageously has (have) a specific surface area per unit of mass (SM) ranging from 200 to 1500 $m^2/g$, preferably from 600 to 1200 $m^2/g$ and better still from 600 to 800 $m^2/g$.

The specific surface area per unit mass may be determined by the nitrogen absorption method, known as the BET (Brunauer-Emmett-Teller) method, described in The Journal of the American Chemical Society, vol. 60, page 309, February 1938, which corresponds to International Standard ISO 5794/1 (appendix D). The BET specific surface area corresponds to the total specific surface area of the particles under consideration.

The aerogel particles that may be used in the present invention advantageously have a size, expressed as the average diameter (D[0.5]) and measured according to the method previously described, of less than 1500 $\mu$m, and preferably ranging from 1 to 30 $\mu$m, preferably from 5 to 25 $\mu$m, better still from 5 to 20 $\mu$m and even better still from 5 to 15 $\mu$m.

The hydrophobic aerogel particles used in the present invention may advantageously have a tapped density p ranging from 0.04 to 0.10 $g/cm^3$ and preferably from 0.05 to 0.08 $g/cm^3$.

In the context of the present invention, this tapped density p may be assessed according to the following protocol, known as the tapped density protocol:

40 g of powder are poured into a measuring cylinder and the cylinder is then placed on a Stav 2003 machine from Stampf Volumeter. The cylinder is then subjected to a series of 2500 tapping actions (this operation is repeated until the difference in volume between two consecutive tests is less than 2%); the final volume Vf of tapped powder is then measured directly on the cylinder. The tapped density is determined by the ratio: mass (m)/Vf, in this instance 40/Vf (Vf being expressed in $cm^3$ and m in g).

According to one embodiment, the hydrophobic aerogel particles used in the present invention have a specific surface area per unit of volume SV ranging from 5 to 60 $m^2/cm^3$, preferably from 10 to 50 $m^2/cm^3$ and better still from 15 to 40 $m^2/cm^3$.

The specific surface area per unit of volume is given by the relationship: SV = SM $\times$ p, where p is the tapped density, expressed in $g/cm^3$, and SM is the specific surface area per unit of mass, expressed in $m^2/g$, as previously defined.

The aerogel particle(s) are preferably particles of aerogel of silylated silica (INCI name: silica silylate), and more particularly particles of aerogels of hydrophobic silica surface-modified with trimethylsilyl groups (trimethylsiloxy silica).

According to one preferred embodiment, the aerogel particle(s) may be chosen from:

- the aerogel sold under the name VM-2260 (INCI name: Silica silylate), by the company Dow Corning, the particles of which have an average size of approximately 1000 microns and a specific surface area per unit of mass ranging from 600 to 800 $m^2/g$;
- the aerogels sold by the company Cabot under the references Aerogel TLD 201, Aerogel OGD 201 and Aerogel TLD 203, Enova Aerogel MT 1100 and Enova Aerogel MT 1200.

According to one preferred embodiment, one (or more) aerogel particle(s) is (are) used as mineral spherical filler in the composition according to the present invention, preferably the aerogel sold under the name VM-2270 (INCI name: Silica silylate), by the company Dow Corning, the particles of which have an average size ranging from 5-15 microns and a specific surface area per unit of mass ranging from 600 to 800 $m^2/g$.

As mineral spherical filler, mention may also be made of silicas such as Sunsil 130 sold by Sunjin Chemical (INCI name: silica) and metal (poly)oxides such as bismuth (poly)oxides.

According to one preferred embodiment of the present invention, the spherical filler(s) is (are) chosen from polyamide powders, polymethyl methacrylate microspheres, polytetrafluoroethylene powders, silicas, aerogel particles and metal (poly)oxides, and mixtures thereof.

According to one preferred embodiment of the present invention, the additional filler(s) is (are) chosen from spherical fillers, preferably chosen from polyamide powders, polymethyl methacrylate microspheres, polytetrafluoroethylene powders, aerogel particles and metal (poly)oxides, and mixtures thereof.

Advantageously, the spherical filler(s) is (are) present in a content of less than or equal to 30% by weight, preferably in a content of less than 20% by weight, relative to the total weight of the composition.

Advantageously, the spherical filler(s) is (are) present in a content of less than or equal to 50% by weight, relative to the total weight of additional filler(s).

◦ Lamellar fillers

As indicated above, lamellar fillers are fillers of parallelepipedal shape (rectangular or square surface), discoid shape (circular surface) or ellipsoid shape (oval surface), characterized by three dimensions: a length, a width and a height. When the shape is circular, the length and the width are identical and correspond to the diameter of a disk, whereas the height corresponds to the thickness of the disk. When the surface is oval, the length and the width correspond, respectively, to the large axis and the small axis of an ellipse and the height corresponds to the thickness of the elliptic disk formed by the platelet. When it is a parallelepiped, the length and the width may be of identical or different dimensions: when they are of the same dimension, the shape of the surface of the parallelepiped is a square; in the contrary case, the shape is rectangular. As regards the height, it corresponds to the thickness of the parallelepiped.

The lamellar fillers used according to the invention have a length ranging from 0.01 to 100 μm, preferably from 0.1 to 50 μm and preferably from 1 to 50 μm. The platelets have a length ranging from 0.01 to 100 μm, preferably from 0.1 to 50 μm and preferably from 1 to 10 μm. The platelets have a height (thickness) ranging from 0.1 nm to 1 μm, preferably from 1 nm to 600 nm and preferably from 1 nm to 500 nm.

As lamellar fillers that may be used in the composition of the invention, mention may be made of lamellar silicates, such as talcs, micas, perlites, and mixtures thereof.

Talcs are hydrated magnesium silicates usually comprising aluminum silicate. The crystal structure of talc consists of repeated layers of a sandwich of brucite between layers of silica. As talcs, mention may in particular be made of the product sold under the name Micro Ace P3 by Nippon talc (INCI name: talc), the product sold under the name Luzenac 00 by Imerys (INCI name: talc), or else the product sold under the name Luzenac Pharma M by Imerys (INCI name: talc).

Micas are aluminum silicates optionally comprising iron and/or alkali metals. They have the property of being able to split up into thin layers (aproximately 1 μm). They generally have a dimension ranging from 5 to 150 μm, preferably from 10 to 100 μm and better still from 10 to 60 μm for the largest dimension (length) and a height (thickness) of from 0.1 to 0.5 μm. Mention may be made, as micas, of phlogopite, muscovite, fluorophlogopite, vermiculite and mixtures thereof. As micas, mention may in particular be made of the product sold under the name sericite S-152-BC by Miyoshi Kasei (INCI name: mica), and Mearlmica treated SVA sold by BASF Personal Care Ingredients (INCI name: mica (and) lauroyl lysine).

Mention may also be made, among lamellar silicates, of perlites and preferably expanded perlites. The perlites which can be used according to the invention are generally aluminosilicates of volcanic origin and have the composition:

- 70.0-75.0% by weight of silica $SiO_2$
- 12.0-15.0% by weight of aluminum oxide $Al_2O_3$
- 3.0-5.0% of sodium oxide $Na_2O$
- 3.0-5.0% of potassium oxide $K_2O$
- 0.5-2% of iron oxide $Fe_2O_3$
- 0.2-0.7% of magnesium oxide MgO
- 0.5-1.5% of calcium oxide CaO
- 0.05-0.15% of titanium oxide $TiO_2$.

The perlite is ground, dried and then calibrated in a first stage. The product obtained, known as perlite ore, is gray-colored and has a size of the order of 100 μm. The perlite ore is subsequently expanded (1000°C/2 seconds) to give more or less white particles. When the temperature reaches 850-900°C, the water trapped in the structure of the material evaporates and brings about the expansion of the material, with respect to its original volume. The expanded perlite particles in accordance with the invention may be obtained via the expansion process described in patent US 5 002 698.

Preferably, the perlite particles used will be ground; in this case, they are known as Expanded Milled Perlite (EMP). They preferably have a particle size defined by a median diameter D50 ranging from 0.5 to 50 μm and preferably from 0.5 to 40 μm.

Preferably, the perlite particles used have an untapped apparent density at 25°C ranging from 10 to 400 kg/$m^3$ (standard DIN 53468) and preferably from 10 to 300 kg/$m^3$.

According to one preferred embodiment of the present invention, the lamellar filler(s) is (are) chosen from talcs

and micas, and mixtures thereof.

According to one preferred embodiment, the additional filler(s) is (are) chosen from lamellar fillers, preferably talcs and micas, and mixtures thereof.

Advantageously, the lamellar filler(s) is (are) present in amounts preferably ranging from 10% to 40% by weight, more preferentially from 20% to 30% by weight, relative to the total weight of the composition.

Advantageously, the lamellar filler(s) is (are) present in a content of greater than 50% by weight, relative to the total weight of additional filler(s).

According to one preferred embodiment, the additional filler(s) is (are) chosen from polyamide powders, organopolysiloxane elastomer powders, metal soaps, silicas, metal (poly)oxides, aerogel particles, talcs, micas, and mixtures thereof, preferably mixtures thereof.

According to one preferred embodiment, the additional filler(s) is (are) chosen from VM-2270 Aerogel Fine Particles sold by Dow Corning (INCI name: Silica Silylate); Micro Ace P3 sold by Nippon talc (INCI name: talc); Sunsil 130 sold by Sunjin Chemical (INCI name: silica); Luzenac 00 salt by Imerys (INCI name: talc); Orgasol 2002 sold by Arkema (INCI name: nylon-12); magnesium stearate sold by Stearineries Dubois; sericite S-152-BC sold by Miyoshi Kasei (INCI name: mica); KSP 100 by the company Shin Etsu (INCI name: vinyl dimethicone/methicone silsesquioxane crosspolymer); and mixtures thereof, preferably mixtures thereof.

- **coloring agent(s)**

The compositions according to the invention may also contain at least one coloring agent.

The coloring agent(s) or colorant(s) according to the invention is (are) preferably chosen from pigments, nacres, water-soluble or liposoluble dyes, and mixtures thereof.

The coloring agent(s) is (are) present in a total content of greater than or equal to 1% by weight, relative to the total weight of the composition.

◦ Pigments

The pigments may be white or colored, and mineral and/or organic.

Among the mineral pigments that may be mentioned are zirconium (poly)oxides, cerium (poly)oxides or titanium (poly)oxides, preferably titanium (poly)oxides, more preferentially titanium dioxide, optionally surface-treated, such as the titanium dioxide sold under the name Hombitan FF Pharma by the company Sachtleben, and also zinc (poly)oxides, iron (black, yellow or red) (poly)oxides or chromium (poly)oxides, manganese violet, ultramarine blue, chromium hydrate and ferric blue, and metal powders, for instance aluminum powder and copper powder, and mixtures thereof. According to one preferred embodiment, the mineral pigments are chosen from iron (poly)oxides and titanium (poly)oxides (such as the titanium dioxide sold under the name Hombitan FF Pharma by the company Sachtleben), optionally surface-treated.

The organic pigments can be chosen from cochineal carmine, organic pigments of azo dyes, anthraquinone dyes, indigoid dyes, xanthene dyes, pyrene dyes, quinoline dyes, triphenylmethane dyes, fluorane dyes, and mixtures thereof.

Among the organic pigments, mention may be made in particular of the D&C certified pigments known under the following names: D&C Blue No. 4, D&C Brown No. 1, D&C Green No. 5, D&C Green No. 6, D&C Orange No. 4, D&C Orange No. 5, D&C Orange No. 10, D&C Orange No. 11, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 33, D&C Red No. 34, D&C Red No. 36, D&C Violet No. 2, D&C Yellow No. 7, D&C Yellow No. 8, D&C Yellow No. 10, D&C Yellow No. 11, FD&C Blue No. 1, FD&C Green No. 3, FD&C Red No. 40, FD&C Yellow No. 5, FD&C Yellow No. 6. The chemical materials corresponding to each of the organic colorants mentioned previously are mentioned in the publication International Cosmetic Ingredient Dictionary and Handbook, 1997 edition, pages 371 to 386 and 524 to 528, published by The Cosmetic, Toiletries and Fragrance Association, the content of which is incorporated into the present patent application by reference.

A composition according to the invention comprises a total content of pigments of at least 1% by weight, relative to the total weight of the composition.

◦ Nacres

Examples of nacres that may be mentioned include nacreous pigments such as titanium mica coated with an iron oxide, mica coated with bismuth oxychloride, titanium mica coated with chromium oxide, and nacreous pigments based on bismuth oxychloride, such as Pearl 2600 UVS sold by Farmaquimia (INCI name: bismuth oxychloride). They may also be mica particles at the surface of which are superposed at least two successive layers of metal oxides and/or of organic colorants, such as Mearlmica treated SVA sold by BASF Personal Care Ingredients (INCI name: mica (and)

lauroyl lysine).

According to one embodiment, the pulverulent phase according to the invention comprises at least one additional filler, in particular a spherical filler, which is organic or mineral, preferably mineral, in particular chosen from metal (poly)oxides, preferably bismuth (poly)oxides, and at least one colorant chosen from nacres, such as nacreous pigments, in particular those based on bismuth oxychloride or coated with bismuth oxychloride, such as the product sold under the name Pearl 2600 UVS by Farmaquimia.

The nacres according to the invention are present in a total content of greater than or equal to 1% by weight, relative to the total weight of the composition.

◦ Dyes

Besides the fillers and pigments, the particulate phase of the invention may comprise water-soluble or liposoluble dyes.

The liposoluble dyes are, for example, Sudan red, D&C Red No. 17, D&C Green No. 6, β-carotene, soybean oil, Sudan Brown, D&C Yellow No. 11, D&C Violet No. 2, D&C Orange No. 5, quinoline yellow, annatto and bromo acids.

As water-soluble dyes that are suitable for use in the invention, mention may be made in particular of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophylline, methylene blue, anthocyanins (enocianin, black carrot, hibiscus and elder), caramel and riboflavin.

Preferably, a composition according to the invention comprises at least one coloring agent chosen from pigments, preferably mineral pigments, in particular from metal (poly)oxides, in particular from iron ,oxides, titanium (poly)oxides, such as the titanium dioxide sold under the name Hombitan FF Pharma by the company Sachtleben, and mixtures thereof, for example present in a content of greater than or equal to 1% by weight relative to the total weight of the composition.

- **insoluble UV-screening agent**

The pulverulent phase may comprise at least one insoluble UV-**screening** agent.

The insoluble UV-screening agent(s) is (are) preferably present in the compositions according to the invention in a total content greater than or equal to 2% by weight, relative to the total weight of the composition, preferably in a content ranging from 5% to 30% by weight, and more particularly from 5% to 10% by weight, relative to the total weight of the composition.

The insoluble UV-screening agent(s) is (are) preferably present in the compositions according to the invention in a total content greater than or equal to 2% by weight, relative to the total weight of the pulverulent phase, preferably in a content ranging from 5% to 40% by weight, and more particularly from 5% to 15% by weight, relative to the total weight of the pulverulent phase.

◦ Insoluble organic UV-screening agent

Among the insoluble organic **UV**-screening agents, mention may be made of those described in patent applications US 5 237 071, US 5 166 355, GB 2 303 549, DE 197 26 184 and EP 893 119, in particular methylenebis(hydroxyphenylbenzotriazole) derivatives such as methylenebis(benzotriazolyl)tetramethylbutylphenol sold in solid form under the trade name Mixxim BB/100 by Fairmount Chemical or in micronized form in aqueous dispersion under the trade name Tinosorb M by BASF.

Mention may also be made of the symmetrical triazine UV-screening agents described in patent US 6 225 467, patent application WO 2004/085412 (see compounds 6 and 9) or the document "Symmetrical Triazine Derivatives" IP.COM Journal, IP.COM INC West Henrietta, NY, US (20 September 2004), in particular 2,4,6-tris(biphenyl)-1,3,5-triazines (in particular 2,4,6-tris(biphenyl-4-yl)-1,3,5-triazine) and 2,4,6-tris(terphenyl)-1,3,5-triazine which is also mentioned in Beiersdorf patent applications WO 06/035 000, WO 06/034 982, WO 06/034 991, WO 06/035 007, WO 2006/034 992 and WO 2006/034 985.

◦ Inorganic UV-screening agents

According to one preferred embodiment, the insoluble UV-screening agent(s) is (are) one (or more) inorganic UV-screening agent(s).

The inorganic UV-screening agents used in accordance with the present invention are metal (poly)oxides.

According to one particular form of the invention, the inorganic UV-screening agent(s) of the invention is (are) one (or more) particle(s) of metal oxides having an average elementary particle size of less than or equal to 0.5 μm,

more preferentially between 0.005 μm and 0.5 μm and even more preferentially between 0.01 μm and 0.1 μm, and preferentially between 0.015 μm and 0.05 μm.

The term "average size" of the particles is intended to mean the parameter D[4,3] measured using a Mastersizer 2000 particle size analyzer (Malvern). The light intensity scattered by the particles as a function of the angle at which they are illuminated is converted to size distribution according to the Mie theory. The parameter D[4,3] is measured; this is the average diameter of the sphere having the same volume as the particle. For a spherical particle, reference will often be made to the "average diameter".

The term "average elementary size" is intended to mean the non-aggregated particle size.

The inorganic UV-screening agent(s) may in particular be chosen from coated or uncoated titanium (poly)oxides, zinc (poly)oxides, iron (poly)oxides, zirconium (poly)oxides and cerium (poly)oxides, and mixtures thereof, and more particularly titanium (poly)oxides. Such coated or uncoated metal oxides are in particular described in patent application EP-A-0 518 773. Commercial pigments that may be mentioned include the products sold by the companies Kemira, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminum salts of fatty acids, metal alkoxides (of titanium or aluminum), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil from the company Ikeda,
- with silica and iron oxide, such as the product Sunveil F from the company Ikeda,
- with silica and alumina, such as the products Microtitanium Dioxide MT 500 SA and Microtitanium Dioxide MT 100 SA from the company Tayca and Tioveil from the company Tioxide,
- with alumina, such as the products Tipaque TTO-55 (B) and Tipaque TTO-55 (A) from the company Ishihara and UVT 14/4 from the company Kemira,
- with alumina and aluminum stearate, such as the products Microtitanium Dioxide MT 100 T, MT 100 TX, MT 100 Z and MT-01 from the company Tayca, the products Solaveil CT-10 W and Solaveil CT 100 from the company Uniqema and the product Eusolex T-AVO from the company Merck,
- with silica, alumina and alginic acid, such as the product MT-100 AQ from the company Tayca,
- with alumina and aluminum laurate, such as the product Microtitanium Dioxide MT 100 S from the company Tayca,
- with iron oxide and iron stearate, such as the product Microtitanium Dioxide MT 100 F from the company Tayca,
- with zinc oxide and zinc stearate, such as the product BR351 from the company Tayca,
- with silica and alumina and treated with a silicone, such as the products Microtitanium Dioxide MT 600 SAS, Microtitanium Dioxide MT 500 SAS or Microtitanium Dioxide MT 100 SAS from the company Tayca,
- with silica, alumina and aluminum stearate and treated with a silicone, such as the product STT-30-DS from the company Titan Kogyo,
- with silica and treated with a silicone, such as the product UV-Titan X 195 from the company Kemira,
- with alumina and treated with a silicone, such as the products Tipaque TTO-55 (S) from the company Ishihara or UV Titan M 262 from the company Kemira,
- with triethanolamine, such as the product STT-65-S from the company Titan Kogyo,
- with stearic acid, such as the product Tipaque TTO-55 (C) from the company Ishihara,
- with sodium hexametaphosphate, such as the product Microtitanium Dioxide MT 150 W from the company Tayca,
- with aluminum hydroxide and with stearic acid, such as the product Microtitanium Dioxide MT-100 TV from the company Tayca.

The titanium oxide pigment can be treated:

- $TiO_2$ treated with octyltrimethylsilane, sold under the trade name T 805 by the company Degussa Silices,
- $TiO_2$ treated with a polydimethylsiloxane, sold under the trade name 70250 Cardre UF TiO2SI3 by the company Cardre,
- anatase/rutile TiO2 treated with a polydimethylhydrogenosiloxane, sold under the trade name Microtitanium Dioxide USP Grade Hydrophobic by the company Color Techniques.

The uncoated titanium oxide pigments are sold, for example, by the company Tayca under the trade names Microtitanium Dioxide MT 500 B or Microtitanium Dioxide MT 600 B, by the company Degussa under the name P 25, by the company Wackher under the name Transparent titanium oxide PW, by the company Miyoshi Kasei under the

name UFTR, by the company Tomen under the name ITS and by the company Tioxide under the name Tioveil AQ.

The uncoated zinc oxide pigments are for example:

- those sold under the name Z-Cote by the company Sunsmart;
- those sold under the name Nanox by the company Elementis;
- those sold under the name Nanogard WCD 2025 by the company Nanophase Technologies.

The coated zinc oxide pigments are for example:

- those sold under the name Zinc Oxide CS-5 by the company Toshibi (ZnO coated with polymethylhydrogenosiloxane);
- those sold under the name Nanogard Zinc Oxide FN by the company Nanophase Technologies (as a 40% dispersion in Finsolv TN, C12-C15 alkyl benzoate);
- those sold under the name Daitopersion ZN-30 and Daitopersion ZN-50 by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO by the company Daikin (ZnO coated with perfluoroalkyl phosphate and copolymer based on perfluoroalkylethyl as a dispersion in cyclopentasiloxane);
- those sold under the name SPD-Z1 by the company Shin-Etsu (ZnO coated with silicone-grafted acrylic polymer, dispersed in cyclodimethylsiloxane);
- those sold under the name Escalol Z100 by the company ISP (alumina-treated ZnO dispersed in an ethylhexyl methoxycinnamate/PVP-hexadecene/methicone copolymer mixture);
- those sold under the name Fuji ZnO-SMS-10 by the company Fuji Pigment (ZnO coated with silica and polymethylsilsesquioxane);
- those sold under the name Nanox Gel TN by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide by the company Rhône-Poulenc.

The uncoated iron oxide pigments are sold, for example, by the company Arnaud under the names Nanogard WCD 2002 (FE 45B), Nanogard Iron FE 45 BL AQ, Nanogard FE 45R AQ and Nanogard WCD 2006 (FE 45R) or by the company Mitsubishi under the name TY-220.

The coated iron oxide pigments are sold, for example, by the company Arnaud under the names Nanogard WCD 2008 (FE 45B FN), Nanogard WCD 2009 (FE 45B 556), Nanogard FE 45 BL 345 and Nanogard FE 45 BL or by the company BASF under the name Transparent Iron Oxide.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A, and also the mixture of titanium dioxide and zinc dioxide coated with alumina, silica and silicone, such as the product M 261 sold by the company Kemira, or coated with alumina, silica and glycerol, such as the product M 211 sold by the company Kemira.

According to the invention, the coated or uncoated titanium oxides are particularly preferred.

According to one particular form of the invention, the insoluble screening agents may consist of composite particles with an average size of between 0.1 and 30 μm comprising a matrix and an inorganic UV-screening agent, the content of inorganic screening agent in a particle ranging from 1% to 70% by weight. These composite particles may be chosen from composite spherical particles and composite lamellar particles, or mixtures thereof.

The spherical and non-spherical screening composite particles used according to the present invention comprise a matrix and an inorganic UV-screening agent. The matrix comprises one or more organic and/or inorganic materials.

The inorganic UV-screening agent is preferentially chosen from metal oxides, preferably titanium oxides, zinc oxides, iron oxides or mixtures thereof, and more particularly from titanium dioxide $TiO_2$, and in particular titanium dioxide coated with aluminum hydroxide and with stearic acid, such as the product Microtitanium Dioxide MT-100 TV from the company Tayca.

According to one particular embodiment, the pulverulent phase according to the invention comprises at least one insoluble UV-screening agent, in particular chosen from metal (poly)oxides, preferably titanium (poly)oxides, and in particular titanium oxides coated with aluminum hydroxide and with stearic acid, such as the product Microtitanium Dioxide MT-100 TV from the company Tayca, and at least one colorant chosen from pigments, in particular from metal (poly)oxides, in particular titanium (poly)oxides, optionally surface-treated, such as the titanium dioxide sold under the name Hombitan FF Pharma by the company Sachtleben, iron (poly)oxides, and mixtures thereof.

These metal oxides can be provided in the form of particles with an average size generally of less than 200

nm. Advantageously, the metal oxide particles used exhibit an average size of less than or equal to 0.1 μm.

These metal oxides can also be provided in the form of layers, preferably multilayers with an average thickness generally of less than 0.2 μm.

According to a first variant, the composite particles contain a matrix comprising an organic and/or inorganic material, in which matrix particles of inorganic UV-screening agent are included. According to this embodiment, the matrix has inclusions and particles of mineral UV-screening agent are placed in the inclusions of the matrix.

According to a second variant, the composite particles contain a matrix made of an organic and/or inorganic material, which matrix is covered with at least one layer of inorganic UV-screening agent which may be connected to the matrix by means of a binder.

According to a third variant, the composite particles contain an inorganic UV-screening agent covered with at least one layer of an organic and/or inorganic material.

The matrix can also be formed of one or more organic or inorganic materials. There may then be a continuous phase of materials, such as an alloy, that is to say, a continuous phase in which the materials can no longer be separated, or a non-continuous phase of materials, for example composed of an organic or inorganic material covered with a layer of another different organic or inorganic material.

According to one variant, in particular when the spherical composite particles comprise a matrix covered with a layer of UV-screening agent, the composite particles may furthermore be covered with an additional coating, in particular chosen from biodegradable or biocompatible materials, lipid materials, for instance surfactants or emulsifiers, polymers, and oxides.

Spherical composite particles:

The inorganic materials that may be used in the matrix of the spherical composite particles according to the present invention may be chosen from the group formed by boron nitride, glass, calcium carbonate, barium sulfate, hydroxyapatite, silica, silicate, magnesium sulfate, magnesium carbonate, aluminum oxide, calcium silicate, calcium phosphate, magnesium oxide and bismuth oxychloride, and mixtures thereof.

The organic materials which can be used to form the matrix are chosen from the group formed by poly(meth)acrylates, polyamides, silicones, polyurethanes, polyethylenes, polypropylenes, polystyrenes, polyhydroxyalkanoates, polycaprolactams, poly(butylene succinate)s, polysaccharides, polypeptides, polyvinyl alcohols, polyvinyl resins, fluoropolymers, waxes, polyesters, polyethers and mixtures thereof.

Preferably, the matrix of the spherical composite particle contains a material or a mixture of materials chosen from:

- $SiO_2$,
- polymethyl methacrylate,
- copolymers of styrene and of a $C_1/C_5$ alkyl (meth)acrylate derivative,
- polyamides, such as nylon.

The composite particles in spherical form are characterized by an average diameter of between 100 nm and 30 μm, preferably between 300 nm and 20 μm and more preferably between 500 μm and 10 μm.

According to a first alternative form, the spherical composite particles comprise a matrix comprising an organic and/or inorganic material, in which matrix particles of inorganic UV screening agent are included.

According to this first alternative form, the particles of inorganic UV screening agent are characterized by an average elementary size generally of less than 0.2 μm. Advantageously, the metal oxide particles used exhibit an average elementary size of less than or equal to 0.1 μm.

As composite particles corresponding to this variant, mention may be made of the products Sunsil TIN 50 and Sunsil TIN 40 sold by the company Sunjin Chemical. These spherical composite particles having an average size between 2 and 7 μm are formed of $TiO_2$ encapsulated in a silica matrix.

Mention may also be made of the following particles:

- spherical composite particles having an average size between 4 and 8 μm, containing $TiO_2$ and $SiO_2$ and having the trade name Eospoly TR sold by the company Creations Couleurs,
- composite particles containing $TiO_2$ and a styrene/alkyl acrylate copolymer matrix sold under the name Eospoly UV TR22 HB 50 by the company Creations Couleurs,
- composite particles containing $TiO_2$ and ZnO and a PMMA matrix and having the trade name Sun PMMA-T50 sold by the company Sunjin Chemical.

According to a second variant, the spherical composite particles contain a matrix made of an organic and/or

inorganic material, covered with at least one layer of inorganic UV-screening agent connected to the matrix by means of a binder.

According to this second variant, the mean thickness of the layer of inorganic UV-screening agent is generally about ten nanometers. The mean thickness of the layer of inorganic UV screening agent is advantageously between $10^{-3}$ and 0.2 μm and preferably between 0.001 and 0.2 μm.

The spherical composite particles used according to the invention have a size of between 0.1 and 30 μm, preferably between 0.3 and 20 μm and even more preferentially between 0.5 and 10 μm.

Among the composite particles that can be used according to the invention, mention is made of spherical composite particles containing $TiO_2$ and $SiO_2$ and having the trade name STM ACS-0050510, sold by the company JGC Catalysts and Chemical.

According to a third variant, the spherical composite particles contain an inorganic UV-screening agent covered with at least one layer of an organic and/or inorganic material. According to this third variant, the particles of inorganic UV screening agent are characterized by an average elementary size generally of between $10^{-3}$ and 0.2 μm. Advantageously, the metal oxide particles used exhibit an average elementary size of between 0.01 and 0.1 μm.

The spherical composite particles used according to the invention have a size of between 0.1 and 30 μm, preferably between 0.3 and 20 μm and more preferentially still between 0.5 and 10 μm.

<u>Non-spherical composite particles:</u>

The organic materials that may be used to form the matrix of the screening non-spherical particles are chosen from the group formed by poly(meth)acrylates, polyamides, silicones, polyurethanes, polyethylenes, polypropylenes, polystyrenes, polyhydroxyalkanoates, polycaprolactams, poly(butylene)succinates, polysaccharides, polypeptides, polyvinyl alcohols, polyvinyl resins, fluoropolymers, waxes, polyesters, polyethers, and mixtures thereof.

Preferably, the organic materials that can be used are:

- triethoxycaprylylsilane,
- acrylic polymers such as polymethyl methacrylate and acrylic copolymers comprising other types of monomers such as styrene;
- polyamides, such as nylon.

The inorganic materials that can be used in the matrix of the non-spherical composite particles are chosen from the group formed by mica, synthetic mica, talc, sericite, boron nitride, glass, calcium carbonate, barium sulfate, hydroxyapatite, silica, silicate, magnesium sulfate, magnesium carbonate, magnesium trisilicate, aluminum oxide, calcium silicate, calcium phosphate, magnesium oxide and bismuth oxychloride, and mixtures thereof. Preferably, these inorganic materials are chosen from:

- silica,
- talc,
- mica,
- alumina.

The inorganic UV-screening agent is generally chosen from metal oxides and in particular from titanium oxides, zinc oxides or iron oxides, which are coated or uncoated, and more particularly titanium dioxide $TiO_2$, in particular titanium dioxide coated with aluminum hydroxide and with stearic acid, such as the product Microtitanium Dioxide MT-100 TV from the company Tayca.

The non-spherical composite particles of the invention are characterized by three dimensions, of which:

- the smallest is greater than 0.1 μm, preferably greater than 0.3 μm and even better still greater than 0.5 μm;
- the largest is less than 30 micrometers, preferably 20 micrometers and even better still 10 micrometers.

The ratio of the largest to the smallest dimension is greater than 1.2.

The dimensions of the particles of the invention are evaluated by scanning electron microscopy and image analysis.

The non-spherical composite particles that may be used according to the invention are preferably platelet-shaped.

The term "platelet-shaped" is intended to mean a parallelepipedal shape.

They may be smooth, rough or porous.

The platelet-shaped composite particles preferably have an average thickness of between 0.01 and 10 μm,

the mean length is generally between 0.5 and 30 μm and the mean width is between 0.5 and 30 μm.

The thickness is the smallest of the dimensions, the width is the medium dimension, and the length is the longest of the dimensions.

According to a first variant, the composite particles contain a matrix comprising an organic and/or inorganic material, in which matrix particles of inorganic UV-screening agent are included. According to this first alternative form, the particles of inorganic UV screening agent are characterized by an average elementary size generally of less than 0.2 μm. Advantageously, the metal oxide particles used have an average elementary size of less than or equal to 0.1 μm.

According to a second variant, the composite particles contain a matrix made of an organic and/or inorganic material, which matrix is covered with at least one layer of inorganic UV-screening agent which may be connected to the matrix by means of a binder.

According to this second variant, the mean thickness of the layer of inorganic UV-screening agent is generally about ten nanometers. The mean thickness of the layer of inorganic UV-screening agent is advantageously between $10^{-3}$ and 0.2 μm and preferably between 0.01 and 0.2 μm.

The non-spherical composite particles used according to the invention have a size of between 100 nm and 30 μm, preferably between 0.3 and 20 μm and more preferentially still between 0.5 and 10 μm.

According to a third variant, the non-spherical composite particles contain an inorganic UV-screening agent covered with at least one layer of an organic and/or inorganic material. According to this third variant, the particles of inorganic UV screening agent are characterized by an average elementary size generally of between $10^{-3}$ and 0.2 μm. Advantageously, the metal oxide particles used exhibit an average elementary size of between 0.01 and 0.1 μm.

Preferably, the inorganic UV-screening agent used in the composite particle is chosen from metal oxides, in particular from titanium oxides, zinc oxides or iron oxides and more particularly titanium dioxide ($TiO_2$).

Preferably, the matrix of the composite particle contains a material or a mixture of materials chosen from $SiO_2$, alumina, mica, the alumina/triethoxycaprylylsilane mixture, talc, PMMA (polymethyl methacrylate) and Nylon.

More preferably, the matrix of the composite particle is formed from a material or mixture of materials chosen from alumina, the alumina/triethoxycaprylylsilane mixture, talc, silica and mica.

Among the composite particles that may be used according to the invention, mention may also be made of the following particles:

- composite particles containing $TiO_2$ and an alumina matrix, having the trade name Matlake OPA AS, sold by the company Sensient LCW,
- composite particles containing $TiO_2$ and an alumina/triethoxycaprylylsilane matrix, having the trade name Matlake OPA AS, sold by the company Sensient LCW,
- composite particles containing ultrafine $TiO_2$ particles deposited on the surface of talc platelets, having the trade name TTC 30, sold by the company Miyoshi Kasei,
- composite particles containing ultrafine $TiO_2$ particles deposited on the surface of talc platelets, having the trade name Silseem Mistypearl Yellow®, sold by the company Nihon Koken Kogyo (NKK).

According to one preferred embodiment of the invention, the insoluble UV-screening agent(s) is (are) one (or more) inorganic UV-screening agent(s), preferably one (or more) metal (poly)oxides, in particular titanium (poly)oxides, in particular titanium oxides coated with aluminum hydroxide and with stearic acid, such as the product Microtitanium Dioxide MT-100 TV from the company Tayca, in a content of greater than or equal to 2% by weight, preferably ranging from 5% to 30% by weight, preferably ranging from 5% to 10% by weight, relative to the total weight of the composition.

According to one preferred embodiment of the invention, the inorganic UV-screening agent(s) is (are) one (or more) metal (poly)oxides, in particular titanium oxides, in particular titanium oxides coated with aluminum hydroxide and with stearic acid, such as the product Microtitanium Dioxide MT-100 TV from the company Tayca, and is (are) present in a content of greater than or equal to 2% by weight, preferably in a content ranging from 5% to 40% by weight, preferably ranging from 5% to 15% by weight, relative to the total weight of the pulverulent phase.

**LIQUID FATTY PHASE:**

The composition according to the invention comprises at least one liquid fatty phase. According to one embodiment, the composition according to the invention comprises a liquid fatty phase in a content ranging from 5% to 45% by weight, preferably ranging from 5% to 40% by weight, better still from 10% to 30% by weight and more preferentially from 15% to 20% by weight, relative to the total weight of the composition.

For the purposes of the present invention, the liquid fatty phase comprises at least one lipophilic organic **UV**-screening agent. The liquid fatty phase may also comprise one (or more) oil(s), for example hydrocarbon-based or silicone oil(s).

According to one particular embodiment, the liquid fatty phase may comprise one or more liquid lipophilic UV-screening agents and in particular can consist essentially, or even solely, of said liquid lipophilic UV screening agent(s).

## Lipophilic organic UV-screening agent

The liquid fatty phase comprises at least one lipophilic organic UV-screening agent.
The lipophilic organic UV-screening agent(s) is (are) chosen in particular from cinnamic derivatives; anthranilates; salicylic derivatives; dibenzoylmethane derivatives, camphor derivatives; benzophenone derivatives; phenyl benzotriazole derivatives; β,β-diphenylacrylate derivatives; triazine derivatives; benzotriazole derivatives; phenyl benzotriazole derivatives; benzalmalonate derivatives, in particular those mentioned in patent US 5 624 663; imidazolines; p-aminobenzoic acid (PABA) derivatives; benzoxazole derivatives as described in patent applications EP 0 832 642, EP 1 027 883, EP 1300 137 and DE 101 62 844; screening polymers and screening silicones such as those described in particular in patent application WO 93/04665; α-alkylstyrene-based dimers, such as those described in patent application DE 198 55 649; 4,4-diarylbutadienes such as those described in patent applications EP 0 967 200, DE 197 46 654, DE 197 55 649, EP-A-1 008 586, EP 1 133 980 and EP 133 981; merocyanin derivatives such as those described in patent applications WO 04/006 878, WO 05/058 269, WO 06/032 741, FR 2 957 249 and FR 2 957 250; and mixtures thereof.
As examples of lipophilic organic UV-screening agents, mention may be made of those denoted hereinbelow under their INCI name:

- Dibenzoylmethane derivative: Butylmethoxydibenzoylmethane or avobenzone sold under the trade name Parsol 1789 by the company DSM Nutritional Products,
- para-Aminobenzoic acid derivatives: PABA, Ethyl PABA, Ethyl Dihydroxypropy PABA, Ethylhexyl Dimethyl PABA sold in particular under the name Escalol 507 by ISP,
- Salicylic derivatives: Homosalate sold under the name Eusolex HMS by Rona/EM Industries, Ethylhexyl Salicylate sold under the name Neo Heliopan OS by Symrise,
- Cinnamic derivatives: Ethylhexyl Methoxycinnamate sold in particular under the trade name Parsol MCX by DSM Nutritional Products., Isopropyl Methoxycinnamate, Isoamylmethoxy cinnamate sold under the trade name Neo Heliopan E 1000 by Symrise, Cinoxate, Diisopropyl Methylcinnamate,
- β,β-Diphenyl acrylate derivatives: Octocrylene sold in particular under the trade name Uvinul N539 by BASF, Etocrylene, sold in particular under the trade name Uvinul N35 by BASF,
- Benzophenone derivatives: Benzophenone-1 sold under the trade name Uvinul 400 by BASF, Benzophenone-2 sold under the trade name Uvinul D50 by BASF, Benzophenone-3 or Oxybenzone, sold under the trade name Uvinul M40 by BASF, Benzophenone-6 sold under the trade name Helisorb 11 by Norquay, Benzophenone-8 sold under the trade name Spectra-Sorb UV-24 by American Cyanamid, Benzophenone-12, n-hexyl 2-(4-diethylamino-2-hydroxybenzoyl)benzoate sold under the trade name Uvinul A + or in the form of a mixture with octylmethoxycinnamate under the trade name Uvinul A + B by BASF, 1,1'-(1,4-piperazinediyl)bis[1-[2-[4-(diethylamino)-2-hydroxybenzoyl]phenyl]methanone (CAS 919803-06-8) in its microniszed or non-micronized form,
- Benzylidenecamphor derivatives: 3-Benzylidenecamphor manufactured under the name Mexoryl SD by Chimex, 4-Methylbenzylidenecamphor sold under the name Eusolex 6300 by Merck, Polyacrylamidomethyl Benzylidenecamphor manufactured under the name Mexoryl SW by Chimex,
- Phenylbenzotriazole derivatives: Drometrizole trisiloxane sold under the name Silatrizole by Rhodia Chimie,
- Triazine derivatives: BisEthylhexyloxyphenol Methoxyphenyl Triazine sold under the trade name Tinosorb S by BASF, Ethylhexyl triazone sold under the trade name Uvinul T150 by BASF, Diethylhexyl Butamido Triazone sold under the trade name Uvasorb HEB by Sigma 3V, 2,4,6-tris(dineopentyl 4'-aminobenzalmalonate)-s-triazine, 2,4,6-tris(diisobutyl 4'-aminobenzalmalonate)-s-triazine, 2,4-bis(dineopentyl 4'-aminobenzalmalonate)-6-(n-butyl 4'-aminobenzoate)-s-triazine,
- Anthranilic derivatives: Menthyl Anthranilate sold under the trade name Neo Heliopan MA by Symrise,
- Imidazoline derivatives: Ethylhexyl Dimethoxybenzylidene Dioxoimidazoline Propionate,
- Benzalmalonate derivatives: Dineopentyl 4'-methoxybenzalmalonate, Polyorganosiloxane containing benzalmalonate functions, for instance Polysilicone-15, sold under the trade name Parsol SLX by DSM,
- 4,4-Diarylbutadiene derivatives: 1,1-dicarboxy(2,2'-dimethylpropyl)-4,4-diphenylbutadiene,
- Benzoxazole derivatives: 2,4-bis[5-(1-dimethylpropyl)benzoxazol-2-yl(4-phenyl)iminol-6-(2-ethylhexyl)imino-1,3,5-triazine sold under the name Uvasorb K2A by Sigma 3V, and mixtures thereof,
- Lipophilic merocyanin derivatives: Octyl 5-N,N-diethylamino-2-phenylsulfonyl-2,4-pentadienoate.

The preferential lipophilic organic UV-screening agent(s) is (are) chosen from butyl methoxy dibenzoylmethane, ethylhexyl methoxycinnamate, ethylhexyl salicylate, homosalate, butyl methoxydibenzoylmethane, octocrylene, benzophenone-3, n-hexyl 2-(4-diethylamino-2-hydroxybenzoyl)benzoate, 4-methylbenzylidenecamphor, bis-ethylhexyloxyphenol methoxyphenyl triazine, ethylhexyl triazone, diethylhexyl butamidotriazone, 2,4,6-tris(dineopentyl 4'-aminobenzalmalonate)-s-triazine, 2,4,6-tris-(diisobutyl 4'-aminobenzalmalonate)-s-triazine, 2,4-bis(dineopentyl 4'-aminobenzalmalonate)-6-(n-butyl 4'-aminobenzoate)-s-triazine, 2,4,6-tris(biphenyl-4-yl)-1,3,5-triazine, 2,4,6-tris(terphenyl)-1,3,5-triazine, polysilicone-15, 1,1-dicarboxy(2,2'-dimethylpropyl)-4,4-diphenylbutadiene, 2,4-bis[5-1(dimethylpropyl)benzoxazol-2-yl(4-phenyl)imino]-6-(2-ethylhexyl)imino-1,3,5-triazine, and mixtures thereof;

The lipophilic organic UV-screening agent(s) is (are) present in the compositions according to the invention in a total content greater than or equal to 0.5% by weight, relative to the total weight of the composition, preferably in a content ranging from 1% to 25% by weight, preferably from 4% to 20% by weight, and more particularly from 7% to 20% by weight, relative to the total weight of the composition.

The lipophilic organic UV-screening agent(s) is (are) present in the compositions according to the invention in a content greater than or equal to 40% by weight, preferably in a content ranging from 50% to 70% by weight, relative to the total weight of the liquid fatty phase;

According to one embodiment, the lipophilic organic screening agent(s) and the inorganic UV-screening agent(s) are present in a respective weight content such that the ratio of the total content of lipophilic organic UV-screening agent(s) to the content of insoluble UV-screening agent(s) ranges from 0 to 10, preferably from 1 to 6.

According to one embodiment, the lipophilic organic UV-screening agent(s) is (are) chosen from cinnamic derivatives; salicylic derivatives; triazine derivatives; phenyl benzotriazole derivatives; and mixtures thereof, and is (are) present in the compositions according to the invention in a total content greater than or equal to 0.5% by weight, relative to the total weight of the composition, preferably in a content ranging from 1% to 25% by weight, preferably from 4% to 20% by weight, and more particularly from 7% to 20% by weight, relative to the total weight of the composition.

According to one embodiment, the lipophilic organic UV-screening agent(s) is (are) chosen from cinnamic derivatives; salicylic derivatives; triazine derivatives; phenyl benzotriazole derivatives; and mixtures thereof, and is (are) present in the compositions according to the invention in a total content greater than or equal to 40% by weight, preferably in a content ranging from 50% to 70% by weight, relative to the total weight of the liquid fatty phase.

According to one embodiment, the lipophilic organic UV-screening agent(s) is (are) chosen from ethylhexyl methoxycinnamate (sold under the name Parsol MCX by DSM Nutritional Products), ethylhexyl salicylate (sold under the name Neo Heliopan OS/H by Symrise), ethylhexyl triazone (sold under the name Uvinul T150 by BASF), bis-ethylhexyloxyphenol methoxyphenyl triazine (sold under the name Tinosorb S by BASF), drometrizole trisiloxane (sold under the name Silatrizole by Rhodia), and mixtures thereof and is (are) present in the compositions according to the invention in a content greater than or equal to 0.5% by weight, relative to the total weight of the composition, preferably in a content ranging from 1% to 25% by weight, in particular from 4% to 20% by weight, and more particularly from 7% to 20% by weight, relative to the total weight of the composition.

According to one embodiment, the lipophilic organic UV-screening agent(s) is (are) chosen from ethylhexyl methoxycinnamate (sold under the name Parsol MCX by DSM Nutritional Products), ethylhexyl salicylate (sold under the name Neo Heliopan OS/H by Symrise), ethylhexyl triazone (sold under the name Uvinul T150 by BASF), bis-ethylhexyloxyphenol methoxyphenyl triazine (sold under the name Tinosorb S by BASF), drometrizole trisiloxane (sold under the name Silatrizole by Rhodia), and mixtures thereof and is (are) present in the compositions according to the invention in a content greater than or equal to 40% by weight, preferably in a content ranging from 50% to 70% by weight, relative to the total weight of the liquid fatty phase.

- **Oil**

The liquid fatty phase according to the present invention may comprise at least one oil.

The oil(s) is (are) present in the composition in a content greater than or equal to 1% by weight, preferably in a content ranging from 1% to 15% by weight, relative to the total weight of the composition.

The liquid fatty phase comprises a total content of oil(s) greater than or equal to 5% by weight, preferably in a total content ranging from 10% to 50% by weight, relative to the total weight of the liquid fatty phase.

The oil(s) is (are) chosen from hydrocarbon-based oil(s), silicone oil(s), and mixtures thereof, preferably mixtures thereof.

The oil(s) may be volatile or nonvolatile.

The oil(s) is (are) preferably chosen from hydrocarbon-based oils, silicone oils and mixtures thereof, preferably mixtures thereof.

◦ Hydrocarbon-based oil

The liquid fatty phase may comprise at least one hydrocarbon-based oil. The hydrocarbon-based oil(s) may be volatile or nonvolatile.

Mention may in particular be made, as nonvolatile hydrocarbon-based oils which can be used according to the invention, of:

- hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheatgerm oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by Stéarineries Dubois or those sold under the names Miglyol 810, 812 and 818 by Dynamit Nobel;
- synthetic ethers containing from 10 to 40 carbon atoms,
- linear or branched hydrocarbons of mineral or synthetic origin, such as petroleum jelly, white mineral liquid paraffin, polydecenes, hydrogenated polyisobutene such as parleam, and squalane, and mixtures thereof;
- synthetic esters, for instance the oils of formula RCOOR' in which R represents a linear or branched fatty acid residue comprising from 1 to 40 carbon atoms and R' represents a hydrocarbon-based chain that is in particular branched, containing from 1 to 40 carbon atoms, on condition that $R + R' \geq 10$, for instance purcellin oil (cetostearyl octanoate), isopropyl myristate, isopropyl palmitate, $C_{12}$-$C_{15}$ alkyl benzoate, for instance the product sold under the trade name Finsolv TN or Witconol TN by the company Witco or Tegosoft TN by the company Evonik Goldschmidt, 2-ethylphenyl benzoate, for instance the commercial product sold under the name X-Tend 226 by the company ISP, isopropyl lanolate, hexyl laurate, diisopropyl adipate, isononyl isononanoate, oleyl erucate, 2-ethylhexyl palmitate, isostearyl isostearate, alcohol or polyalcohol octanoates, decanoates or ricinoleates, for instance propylene glycol dioctanoate; hydroxylated esters, for instance isostearyl lactate, diisostearyl malate; and pentaerythritol esters; citrates or tartrates, for instance linear $C_{12}$-$C_{13}$ dialkyl tartrates, such as those sold under the name Cosmacol ETI by the company Enichem Augusta Industriale, and also linear $C_{14}$-$C_{15}$ dialkyl tartrates such as those sold under the name Cosmacol ETL by the same company; acetates;
- fatty alcohols that are liquid at ambient temperature, with a branched and/or unsaturated carbon-based chain containing from 12 to 26 carbon atoms, for instance octyldodecanol, isostearyl alcohol, oleyl alcohol, 2-hexyldecanol, 2-butyloctanol and 2-undecylpentadecanol;
- higher fatty acids such as oleic acid, linoleic acid or linolenic acid;
- carbonates, such as dicaprylyl carbonate, such as the product sold under the name Cetiol CC by the company Cognis;
- fatty amides, for instance isopropyl N-lauroyl sarcosinate, for instance the product sold under the trade name Eldew SL205 from Ajinomoto;
- and mixtures thereof.

As volatile hydrocarbon-based oils that may be used according to the invention, mention may be made in particular of hydrocarbon-based oils containing from 8 to 16 carbon atoms, and in particular branched $C_8$-$C_{16}$ alkanes such as $C_8$-$C_{16}$ isoalkanes of petroleum origin (also known as isoparaffins), for instance isododecane (also known as 2,2,4,4,6-pentamethylheptane), isodecane, isohexadecane, and the alkanes described in the patent applications from the company Cognis WO 2007/068 371 or WO 2008/155 059 (mixtures of different alkanes differing by at least one carbon). These alkanes are obtained from fatty alcohols, which are themselves obtained from coconut oil or palm oil, the oils sold under the trade name Isopar or Permethyl, branched $C_8$-$C_{16}$ esters isohexyl neopentanoate, and mixtures thereof.

Other volatile hydrocarbon-based oils, for instance petroleum distillates, in particular those sold under the name Shell Solt by the company Shell, may also be used. According to one embodiment, the volatile solvent is chosen from volatile hydrocarbon-based oils containing from 8 to 16 carbon atoms, and mixtures thereof.

According to one preferred embodiment, the hydrocarbon-based oil(s) is (are) chosen from one (or more) nonvolatile oil(s), in particular from synthetic esters, for instance the oils of formula RCOOR' in which R represents a linear or branched fatty acid residue comprising from 1 to 40 carbon atoms and R' represents a hydrocarbon-based chain, which is in particular branched, containing from 1 to 40 carbon atoms, with the proviso that $R + R' \geq 10$, and linear or branched hydrocarbons of mineral or synthetic origin.

In one preferred embodiment, the hydrocarbon-based oil(s) is (are) chosen from one (or more) nonvolatile oil(s), in particular from isononyl isononanoate, petroleum jelly and mineral oil.

◦ Silicone oil

The liquid fatty phase may comprise at least one silicone oil. The silicone oil(s) may be volatile or nonvolatile.

The nonvolatile silicone oils may be chosen in particular from nonvolatile polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Volatile silicone oils that may be mentioned, for example, include volatile linear or cyclic silicone oils, in particular those with a viscosity ≤ 8 centistokes ($8\times10^{-6}$ $m^2/s$) and in particular containing from 2 to 7 silicon atoms, these silicones optionally comprising alkyl or alkoxy groups containing from 1 to 10 carbon atoms. As volatile silicone oil that may be used in the invention, mention may be made in particular of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of volatile linear alkyltrisiloxane oils of general formula (I):

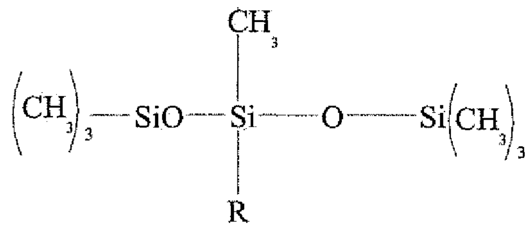

where R represents an alkyl group comprising from 2 to 4 carbon atoms, one or more hydrogen atoms of which may be replaced with a fluorine or chlorine atom.

Among the oils of general formula (I), mention may be made of:

- 3-butyl-1,1,1,3,5,5,5-heptamethyltrisiloxane,
- 3-propyl-1,1,1,3,5,5,5-heptamethyltrisiloxane, and
- 3-ethyl-1,1,1,3,5,5,5-heptamethyltrisiloxane,

corresponding to the oils of formula (I) for which R is respectively a butyl group, a propyl group or an ethyl group.

In one preferred embodiment, the silicone oil(s) is (are) chosen from nonvolatile silicone oils, preferably from nonvolatile silicone oils of cyclic or linear, preferably linear, polydimethylsiloxane type, and more particularly chosen from polydimethylsiloxane having a viscosity of at least 8 cSt, preferably at least 10 cSt.

In one preferred embodiment, the silicone oil(s) is (are) chosen from volatile silicone oils, preferably from cyclic or linear volatile silicone oils of polydimethylsiloxane type, which is preferably linear, and more preferentially chosen from polydimethylsiloxane having a viscosity of less than 8 cSt, preferably of less than 5 cSt.

According to one preferred embodiment, the cyclic or linear, volatile or nonvolatile silicone oil(s), of polydimethylsiloxane type, which is preferably linear, is (are) more preferentially chosen from polydimethylsiloxane.

According to one preferred embodiment, the oil(s) is (are) chosen from volatile or nonvolatile, hydrocarbon-based and/or silicone oil(s), in particular from synthetic esters, for instance the oils of formula RCOOR' in which R represents a linear or branched fatty acid residue comprising from 1 to 40 carbon atoms and R' represents a hydrocarbon-based chain, which is in particular branched, containing from 1 to 40 carbon atoms, with the proviso that R + R'nd more ≥ 10, linear or branched hydrocarbons of mineral or synthetic origin, and silicones of polydimethylsiloxane type, which is preferably linear, and more preferentially chosen from polydimethylsiloxane, and mixtures thereof, and is (are) present in a content greater than or equal to 1% by weight, preferably ranging from 1% to 15% by weight, relative to the total weight of the composition.

According to one preferred embodiment, the silicone oil(s) is (are) chosen from volatile or nonvolatile, hydrocarbon-based and/or silicone oil(s), in particular from synthetic esters, for instance the oils of formula RCOOR' in which R represents a linear or branched fatty acid residue comprising from 1 to 40 carbon atoms and R' represents a hydrocarbon-based chain, which is in particular branched, containing from 1 to 40 carbon atoms, with the proviso that R + R' and more ≥ 10, linear or branched hydrocarbons of mineral or synthetic origin, and silicones of polydimethylsiloxane type, which is preferably linear, a preferentially chosen from polydimethylsiloxane, and mixtures thereof, and is (are) present in a content greater than or equal to 1% by weight, preferably ranging from 1% to 15% by weight, relative to the total weight of the liquid fatty phase.

- **Adjuvants**

The composition may also comprise one or more adjuvant(s).

The adjuvant(s) may be chosen from waxes, thickeners, softeners, humectants, opacifiers, stabilizers, emollients, silicones, fragrances, preservatives, active agents, polymers, surfactants or any other ingredient normally used in the cosmetics and/or dermatological fields.

Needless to say, those skilled in the art will take care to choose the abovementioned optional additional compound(s) and/or the amounts thereof so that the advantageous properties intrinsically attached to the compositions in accordance with the invention are not, or not substantially, detrimentally affected by the envisaged addition(s). Those skilled in the art will choose said active agents according to the effect desired on the skin, hair, eyelashes, eyebrows or nails.

## ASSEMBLY

The present invention also relates to a cosmetic assembly comprising:

- a container delimiting one or more compartments, said compartment being optionally closed by a closing member and optionally not being leaktight, and
- a makeup and/or care composition in accordance with the invention placed inside said compartment(s).

The compartment may be, for example, in the form of a box.

## PROCESS

The present invention also relates to a non-therapeutic cosmetic process for coating and in particular for making up keratin materials. The process according to the invention comprises at least the application, to the surface of the keratin material, in particular the skin, in particular of the face, of at least one composition as previously defined.

## USE

The cosmetic compositions according to the invention find their application in a large number of uses, in particular cosmetic and non-therapeutic uses, for the skin, the lips and the hair, preferably the skin, and in particular of the face.
Another subject of the present invention consists of the use of the compositions according to the invention as defined above in the manufacture of products for the cosmetic treatment of the skin, lips, nails, hair, eyelashes, eyebrows and/or scalp, in particular care products, anti-sun products and makeup products.
According to one particular embodiment, the compositions according to the invention are used as a makeup product, and in particular as a foundation, and as an anti-sun product.

## EXAMPLES

Several examples aiming toillustrate the invention are described in detail hereinafter.
Examples 1 to 5 aim to demonstrate the importance of the presence of a filler or fillers with a wet point and an average size of specific particles in two compositions in accordance with the invention from the viewpoint of three comparative compositions outside the invention.

| | | | example | | | | |
|---|---|---|---|---|---|---|---|
| | | | 1 (according to the invention) | 2 (according to the invention) | 3 (outside the invention) | 4 (outside the invention) | 5 (outside the invention) |
| Powder phase | D50 >15 - Wp > 70 mL/100g | Sumicos Velvet mica 43037 (Sudarshan Chemicals) | 31.1 | - | - | - | - |
| | | Mearlmica SV (BASF Personal care ingredients) | - | 31.1 | - | - | - |
| | D50 >15 - Wp < 70 mL/100g | Mica (and) . dimethicone SA-S-100 (Miyoshi Kasei) | - | - | 31.1 | - | - |
| | D50 <15 - Wp > 70 mL/100g | Mica concord 1000 (sciama) | - | - | - | 31.1 | - |
| | D50 <15- Wp < 70 mL/100g | Sericite S-152-BC (Miyoshi Kasei) | - | - | - | - | 31.1 |
| | additional fillers | | 30 | 30 | 30 | 30 | 30 |
| | pigments | | 17.8 | 17.8 | 17.8 | 17.8 | 17.8 |
| | inorganic screening agents | | 5 | 5 | 5 | 5 | 5 |
| Liquid phase | lipophilic organic screening agents | | 9.5 | 9.5 | 9.5 | 9.5 | 9.5 |
| | binder | | 6.6 | 6.6 | 6.6 | 6.6 | 6.6 |
| | | Total | 100 | 100 | 100 | 100 | 100 |

*Preparation process:*

The powder phase is stirred in a Baker mixer-disperser until it is homogeneous (paddles: 2700 rpm for 5 min, decaker: 3000 rpm for 3 min 30 s).

The liquid phase is heated at 75°C with magnetic stirring until it is homogeneous.

The liquid phase is introduced into the fillers + pigments phase in the Baker tank with stirring (paddles 2700 rpm for 5 min).

The resulting powder is then passed through an Alpine pin mill at a flow rate of 2.4 kg/h and at a rotational speed of 18 000 rpm. The powder is then sieved at 250 μm.

*Evaluation protocol: disintegration, spreading uniformity and cosmeticity*

- for each composition, 2 series of movements are performed: 5 circular movements are performed with a finger or with a sponge, and
- 1 movement for removing the product is performed on a cotton cloth.

Following these movements, the product is observed. A product does not disintegrate when the surface of the product waxes, thus preventing the product from being taken up.

*Results*

The compositions of examples 1 and 2 disintegrate. These compositions are easy to apply, are pleasant on application, spread well, and are comfortable and uniform.

The compositions of examples 3 to 5 do not disintegrate.

Two other examples of compositions according to the invention were subsequently prepared for the purpose of evaluating the SPF result.

| | | | Examples | |
|---|---|---|---|---|
| Phases | Ingredients | | 6 | 7 |
| Pulverulent phase | Filler(s) according to the invention | Sumicos Velvet mica 43037 (Sudarshan Chemicals) D50 >15 - Wp > 70 mL/ 100g | 31.1 | 19 |
| | Additional filler(s) and nacre(s) | Talc (Micro Ace P3, Nippon talc) | 20 | - |
| | | Talc (Luzenac 00, Imerys) | - | 20.9 |
| | | Mica (Sericite S-152-BC, Miyoshi kasei) | - | 15 |
| | | Magnesium stearate DUB SMG (Stearineries Dubois) | - | 2 |
| | | Lauroyl lysine-coated mica (Mearlmica treated SVA, BASF Personal Care Ingredients) | 10 | 15 |
| | Pigment(s) | iron oxides | 7.8 | 6.6 |
| | | titanium dioxide (Hombitan FF Pharma, Sachtleben) | 10 | - |
| | Inorganic screening agent (s) | titanium dioxide and aluminum hydroxide and stearic acid. (Micro titanium dioxide MT-100 TV, Tayca) | 5 | 3 |
| Liquid phase | Lipophilic organic screening agent(s) | ethylhexyl methoxycinnamate (Parsol MCX, DSM Nutritional Products) | 6.5 | 6.5 |
| | | ethylhexyl salicylate (Neo Heliopan OS/H, Symrise) | - | 5 |
| | | ethylhexyl triazone (Uvinul T150, BASF) | - | 1 |
| | | Bisethylhexyloxyphenol methoxyphenyl triazine (Tinosorb S, BASF) | 1 | 1 |
| | | drometrizole trisiloxane (Silatrizole, Rhodia) | 2 | 2 |
| | Binder | $C_{12}$-$C_{15}$ alkyl benzoate (Tegosoft TN ,Evonik Goldschmidt) | 4.5 | - |
| | | mineral oil (Marcol 82, Exxonmobil Chemical) | - | 1.44 |
| | | dimethicone 10 cst (Xiameter PMX-200 Silicone Fluid 5 cs, Dow Corning) | 1.5 | - |
| | | dimethicone 5 cst (Xiameter PMX-200 Silicone Fluid 10 cs, Dow Corning) | - | 1.06 |
| | | preservative | qs | qs |
| | | total | 100 | 100 |

These compositions were produced according to the same preparation process described in example 1.

*Process for evaluating the sun protection factor*

The Sun Protection Factor (SPF) of a product is evaluated according to the International Method published by COLIPA /CTFA SA /JCIA (May 2006).

The Sun Protection Factor is the ratio of the Minimal Erythemal Dose obtained in the presence of product (2

$mg/cm^2$) (MEDp) to the Minimal Erythemal Dose obtained without product (MEDnp).

$$SPF = MEDp/MEDnp$$

The Minimum Erythemal Dose is defined as being the amount of energy necessary to produce the first unambiguous perceptible redness, with clearly defined contours, evaluated 16 to 24 hours after exposure to a sun simulator, at six increasing doses of UV (12% increments).

The test has to be carried out on at least 10 and no more than 20 subjects, and it has to satisfy the statistical criterion (95% CI < 17% mean SPF).

*Result*

The sun protection factors are respectively 37 and 27 for the compositions of examples 6 and 7. The effectiveness (SPF) of compositions 6 and 7 according to the invention is therefore good.

Furthermore, the cosmetic properties of the composition (disintegration, comfort and uniformity) remain excellent. To the knowledge of the inventors, a solid composition having both a high sun protection factor and good cosmetic properties has never been achieved.

Throughout the application, the wording "comprising one" or "containing one" means "comprising at least one" or "containing at least one", unless otherwise specified.

Throughout the application, the contents are expressed in terms of solids.

**Claims**

**1.** A composition, preferably a solid composition, in particular in the form of a loose or compact powder, comprising:

a) at least one pulverulent phase present in a content greater than or equal to 50% by weight relative to the total weight of the composition,
b) at least one liquid fatty phase comprising at least one lipophilic organic UV-screening agent, in which the pulverulent phase comprises at least one filler having an oil absorption capacity, measured at the wet point, of greater than or equal to 70 ml/100 g and a size expressed as volume-average diameter of greater than or equal to 15 microns; the said filler being chosen from the family of micas, in particular natural or synthetic micas; and
c) optionally at least one additional filler having either an oil absorption capacity, measured at the wet point, strictly less than 70 ml/100 g, or a size expressed as volume-average diameter strictly less than 15 microns, or both;

the sizes expressed as volume-average diameter of filler particles being measured by static light scattering using a commercial particle size analyzer.

**2.** The composition as claimed in claim 1, wherein the filler(s) having an oil absorption capacity, measured at the wet point, of greater than or equal to 70 ml/100 g and a size expressed as volume-average diameter of greater than or equal to 15 microns is (are) present according to a total content greater than or equal to 5% by weight, relative to the total weight of the composition, preferably ranging from 10% to 50% by weight, in particular from 20% to 40% by weight, and better still from 25% 35% by weight, relative to the total weight of the composition.

**3.** The composition as claimed in claim 1 or 2, wherein the filler(s) having an oil absorption capacity, measured at the wet point, of greater than or equal to 70 ml/100 g and a size expressed as volume-average diameter of greater than or equal to 15 microns is (are) present according to a total content greater than or equal to 5% by weight, relative to the total weight of the pulverulent phase, preferably ranging from 15% to 60% by weight, in particular from 25% to 50% by weight, and better still from 30% 45% by weight, relative to the total weight of the pulverulent phase.

**4.** The composition as claimed in any one of the preceding claims, wherein the pulverulent phase comprises at least one additional filler, preferably the additional filler(s) is (are) present according to a content ranging from 15% to 60% by weight, relative to the total weight of the pulverulent phase, preferably ranging from 25% to 50% by weight, in particular from 30% to 45% by weight, relative to the total weight of the pulverulent phase, preferably the additional filler(s) is (are) present in a total content greater than or equal to 5% by weight, relative to the total weight of the composition, preferably ranging from 10% to 50% by weight, in particular from 20% to 40% by weight, relative to

the total weight of the composition.

5. The composition as claimed in the preceding claim, wherein the additional filler(s) is (are) chosen from polyamide powders, organopolysiloxane elastomer powders, metal soaps, silicas, aerogel particles, metal (poly)oxides, talcs, micas, and mixtures thereof.

6. The composition as claimed in any one of the preceding claims, wherein the lipophilic organic UV-screening agent(s) is (are) present in a total content greater than or equal to 0.5% by weight, relative to the total weight of the composition, in particular ranging from 1% to 25% by weight relative to the total weight of the composition, preferably ranging from 4% to 20% by weight, even better still from 7% to 20% by weight, relative to the total weight of the composition.

7. The composition as claimed in any one of the preceding claims, wherein the lipophilic organic UV-screening agent(s) is (are) chosen from cinnamic derivatives; anthranilates; salicylic derivative, dibenzoylmethane derivatives, camphor derivatives; benzophenone derivatives; β,β-diphenyl acrylate derivatives; triazine derivatives; benzotriazole derivatives; phenyl benzotriazole derivatives; benzalmalonate derivatives; imidazolines; p-aminobenzoic acid derivatives; benzoxazole derivatives; screening polymers and screening silicones; α-alkylstyrene-derived dimers; 4,4-diarylbutadienes; merocyanine derivatives and mixtures thereof, preferably comprising at least one cinnamic derivative, such as ethylhexyl methoxycinnamate, at least one triazine derivative, such as bis-ethylhexyloxyphenol methoxyphenyl triazine or ethylhexyl triazone, at least one phenyl benzotriazole derivative, such as drometrizole trisiloxane.

8. The composition as claimed in any one of the preceding claims, wherein the lipophilic organic UV-screening agent(s) is (are) chosen from butyl methoxy dibenzoylmethane, ethylhexyl methoxycinnamate, ethylhexyl salicylate, homosalate, butyl methoxydibenzoylmethane, octocrylene, benzophenone-3, n-hexyl 2-(4-diethylamino-2-hydroxybenzoyl)benzoate, 4-methylbenzylidenecamphor, bis-ethylhexyloxyphenol methoxyphenyl triazine, ethylhexyl triazone, diethylhexyl butamido triazone, 2,4,6-tris(dineopentyl 4'-amino benzalmalonate)-s-triazine, 2,4,6-tris(diisobutyl 4'-aminobenzalmalonate)-s-triazine, 2,4-bis(dineopentyl 4'-aminobenzalmalonate)-6-(n-butyl 4'-aminobenzoate)-s-triazine, 2,4,6-tris(biphenyl-4-yl)-1,3,5-triazine, 2,4,6-tris(terphenyl)-1,3,5-triazine, drometrizole trisiloxane, polysilicone-15, 1,1-dicarboxy-(2,2'-dimethylpropyl)-4,4-diphenylbutadiene, 2,4-bis[5-1-(dimethylpropyl)benzoxazol-2-yl-(4-phenyl)imino]-6-(2-ethylhexyl)imino-1,3,5-triazine, and mixtures thereof.

9. The composition as claimed in any one of the preceding claims, wherein the liquid fatty phase comprises at least one oil, preferably nonvolatile oil, in particular chosen from hydrocarbon-based oils, silicone oils, and mixtures thereof.

10. The composition as claimed in the preceding claim, wherein the total content of oil(s) is greater than or equal to 1% by weight, relative to the total weight of the composition, and in particular ranges from 1% to 15% by weight, relative to the total weight of the composition.

11. The composition as claimed in any one of the preceding claims, wherein the pulverulent phase comprises at least one insoluble UV-screening agent, in particular chosen from metal (poly)oxides, in particular titanium (poly)oxide(s), preferably the insoluble UV-screening agent(s) is (are) present in a total content greater than or equal to 2% by weight, relative to the total weight of the composition, in particular ranging from 5% to 30% by weight, preferably from 5% to 10% by weight, relative to the total weight of the composition.

12. The composition as claimed in any one of the preceding claims, wherein the pulverulent phase comprises at least one colorant, in particular chosen from pigments, nacres, dyes, and mixtures thereof.

**Patentansprüche**

1. Zusammensetzung, vorzugsweise feste Zusammensetzung, insbesondere in Form eines losen oder kompakten Pulvers, umfassend:

   a) mindestens eine pulverförmige Phase, die in einem Gehalt größer oder gleich 50 Gew.-%, bezogen auf das Gesamtgewicht der Zusammensetzung, vorliegt,
   b) mindestens eine flüssige Fettphase, die mindestens eine lipophile organische UV-Filtersubstanz umfasst, wobei die pulverförmige Phase mindestens einen Füllstoff mit einer am Wet Point gemessenen Ölabsorptionskapazität größer oder gleich 70 ml/100 g und einer als volumenmittlerer Durchmesser ausgedrückten Größe größer oder gleich 15 Mikron umfasst; wobei der Füllstoff aus der Familie der Glimmer, insbesondere natürlicher

oder synthetischer Glimmer, ausgewählt ist; und

c) gegebenenfalls mindestens einen zusätzlichen Füllstoff mit einer am Wet Point gemessenen Ölabsorptionskapazität von streng weniger als 70 ml/100 g und/oder einer als volumenmittlerer Durchmesser ausgedrückten Größe von streng weniger als 15 Mikron;

wobei die als volumenmittlerer Durchmesser ausgedrückten Größen von Füllstoffteilchen durch statische Lichtstreuung mit einem kommerziellen Teilchengrößenanalysator gemessen werden.

2. Zusammensetzung nach Anspruch 1, wobei der Füllstoff bzw. die Füllstoffe mit einer am Wet Point gemessenen Ölabsorptionskapazität größer oder gleich 70 ml/100 g und einer als volumenmittlerer Durchmesser ausgedrückten Größe größer oder gleich 15 Mikron gemäß einem Gesamtgehalt größer oder gleich 5 Gew.-%, bezogen auf das Gesamtgewicht der Zusammensetzung, vorzugsweise im Bereich von 10 bis 50 Gew.-%, insbesondere von 20 bis 40 Gew.-% und noch besser von 25 bis 35 Gew.-%, bezogen auf das Gesamtgewicht der Zusammensetzung, vorliegt bzw. vorliegen.

3. Zusammensetzung nach Anspruch 1 oder 2, wobei der Füllstoff bzw. die Füllstoffe mit einer am Wet Point gemessenen Ölabsorptionskapazität größer oder gleich 70 ml/100 g und einer als volumenmittlerer Durchmesser ausgedrückten Größe größer oder gleich 15 Mikron gemäß einem Gesamtgehalt größer oder gleich 5 Gew.-%, bezogen auf das Gesamtgewicht der pulverförmigen Phase, vorzugsweise im Bereich von 15 bis 60 Gew.-%, insbesondere von 25 bis 50 Gew.-% und noch besser von 30 bis 45 Gew.-%, bezogen auf das Gesamtgewicht der pulverförmigen Phase, vorliegt bzw. vorliegen.

4. Zusammensetzung nach einem der vorhergehenden Ansprüche, wobei die pulverförmige Phase mindestens einen zusätzlichen Füllstoff umfasst, wobei der zusätzliche Füllstoff bzw. die zusätzlichen Füllstoffe vorzugsweise gemäß einem Gehalt im Bereich von 15 bis 60 Gew.-%, bezogen auf das Gesamtgewicht der pulverförmigen Phase, vorzugsweise im Bereich von 25 bis 50 Gew.-%, insbesondere von 30 bis 45 Gew.-%, bezogen auf das Gesamtgewicht der pulverförmigen Phase, vorliegt bzw. vorliegen, wobei der zusätzliche Füllstoff bzw. die zusätzlichen Füllstoffe vorzugsweise in einem Gesamtgehalt größer oder gleich 5 Gew.-%, bezogen auf das Gesamtgewicht der Zusammensetzung, vorzugsweise im Bereich von 10 bis 50 Gew.-%, insbesondere von 20 bis 40 Gew.-%, bezogen auf das Gesamtgewicht der Zusammensetzung, vorliegt bzw. vorliegen.

5. Zusammensetzung nach dem vorhergehenden Anspruch, wobei der zusätzliche Füllstoff bzw. die zusätzlichen Füllstoffe aus Polyamid-Pulvern, Organopolysiloxanelastomer-Pulvern, Metallseifen, Siliciumdioxiden, Aerogel-Teilchen, Metall(poly)-oxiden, Talken, Glimmern und Mischungen davon ausgewählt ist bzw. sind.

6. Zusammensetzung nach einem der vorhergehenden Ansprüche, wobei die lipophile organische UV-Filtersubstanz bzw. die lipophilen organischen Filtersubstanzen in einem Gesamtgehalt größer oder gleich 0,5 Gew.-%, bezogen auf das Gesamtgewicht der Zusammensetzung, insbesondere im Bereich von 1 bis 25 Gew.-%, bezogen auf das Gesamtgewicht der Zusammensetzung, vorzugsweise im Bereich von 4 bis 20 Gew.-%, noch besser von 7 bis 20 Gew.-%, bezogen auf das Gesamtgewicht der Zusammensetzung, vorliegt bzw. vorliegen.

7. Zusammensetzung nach einem der vorhergehenden Ansprüche, wobei die lipophile organische UV-Filtersubstanz bzw. die lipophilen organischen Filtersubstanzen aus Zimtsäurederivaten; Anthranilaten; Salicylsäurederivaten; Dibenzoylmethanderivaten; Campherderivaten; Benzophenonderivaten; $\beta$,$\beta$-Diphenylacrylatderivaten; Triazinderivaten; Benzotriazolderivaten; Phenylbenzotriazolderivaten; Benzalmalonatderivaten; Imidazolinen; p-Aminobenzoesäurederivaten; Benzoxazolderivaten; Filterpolymeren und Filtersilikonen; von $\alpha$-Alkylstyrol abgeleiteten Dimeren; 4,4-Diarylbutadienen; Merocyaninderivaten und Mischungen davon, die vorzugsweise mindestens ein Zimtsäurederivat, wie Ethylhexylmethoxycinnamat, mindestens ein Triazinderivat, wie Bis(ethylhexyloxyphenol)methoxyphenyltriazin oder Ethylhexyltriazon, mindestens ein Phenylbenzotriazolderivat, wie Drometrizoltrisiloxan, umfassen, ausgewählt ist bzw. sind.

8. Zusammensetzung nach einem der vorhergehenden Ansprüche, wobei die lipophile organische UV-Filtersubstanz bzw. die lipophilen organischen UV-Filtersubstanzen aus Butylmethoxydibenzoylmethan, Ethylhexylmethoxycinnamat, Ethylhexylsalicylat, Homosalat, Butylmethoxydibenzoylmethan, Octocrylen, Benzophenon-3, n-Hexyl-2-(4-diethylamino-2-hydroxybenzoyl)benzoat, 4-Methylbenzylidencampher, Bis(ethylhexyloxyphenol)methoxyphenyltriazin, Ethylhexyltriazon, Diethylhexylbutamidotriazon, 2,4,6-Tris(dineopentyl-4'-aminobenzalmalonat)-s-triazin, 2,4,6-Tris(diisobutyl-4'-aminobenzalmalonat)-s-triazin, 2,4-Bis(dineopentyl-4'-aminobenzalmalonat)-6-(n-butyl-4'-aminobenzoat)-s-triazin, 2,4,6-Tris(biphenyl-4-yl)-1,3,5-triazin, 2,4,6-Tris(terphenyl)-1,3,5-triazin, Drometrizoltrisiloxan, Polysilicone-15, 1,1-Dicarboxy-(2,2'-dimethylpropyl)-4,4-diphenylbutadien, 2,4-Bis[5-1-(dimethylpropyl)benzoxazol-2-yl-(4-phenyl)imino]-6-(2-ethylhexyl)imino-1,3,5-triazin und Mischungen davon ausgewählt ist bzw. sind.

**9.** Zusammensetzung nach einem der vorhergehenden Ansprüche, wobei die flüssige Fettphase mindestens ein Öl, vorzugsweise ein nichtflüchtiges Öl, das insbesondere aus auf Kohlenwasserstoff basierenden Ölen, Silikonölen und Mischungen davon ausgewählt ist, umfasst.

**10.** Zusammensetzung nach dem vorhergehenden Ansprüche, wobei der Gesamtgehalt an Öl bzw. Ölen größer oder gleich 1 Gew.-%, bezogen auf das Gesamtgewicht der Zusammensetzung, ist und insbesondere im Bereich von 1 bis 15 Gew.-%, bezogen auf das Gesamtgewicht der Zusammensetzung, liegt.

**11.** Zusammensetzung nach einem der vorhergehenden Anspruch, wobei die pulverförmige Phase mindestens eine unlösliche UV-Filtersubstanz umfasst, die insbesondere aus Metall(poly)oxiden, insbesondere Titan(poly)oxid bzw. Titan(poly)oxiden, ausgewählt ist, wobei die unlösliche UV-Filtersubstanz bzw. die unlöslichen UV-Filtersubstanzen vorzugsweise in einem Gesamtgehalt größer oder gleich 2 Gew.-%, bezogen auf das Gesamtgewicht der Zusammensetzung, insbesondere im Bereich von 5 bis 30 Gew.-%, vorzugsweise von 5 bis 10 Gew.-%, bezogen auf das Gesamtgewicht der Zusammensetzung, vorliegt bzw. vorliegen.

**12.** Zusammensetzung nach einem der vorhergehenden Ansprüche, wobei die pulverförmige Phase mindestens ein Farbmittel umfasst, das insbesondere aus Pigmenten, Perlmutten, Farbstoffen und Mischungen davon ausgewählt ist.

**Revendications**

**1.** Composition, de préférence composition solide, en particulier sous la forme d'une poudre libre ou compacte, comprenant :

a) au moins une phase pulvérulente présente à une teneur supérieure ou égale à 50 % en poids par rapport au poids total de la composition,
b) au moins une phase grasse liquide comprenant au moins un agent de protection contre les UV organique lipophile, dans laquelle la phase pulvérulente comprend au moins une charge ayant une capacité d'absorption d'huile, mesurée au point de mouillage, supérieure ou égale à 70 ml/100 g et une taille exprimée en diamètre moyen en volume supérieure ou égale à 15 microns ; ladite charge étant choisie dans la famille des micas, en particulier des micas naturels ou synthétiques ; et
c) facultativement, au moins une charge supplémentaire ayant soit une capacité d'absorption d'huile, mesurée au point de mouillage, strictement inférieure à 70 ml/100 g, ou une taille exprimée en diamètre moyen en volume strictement inférieur à 15 microns, ou les deux ;

les tailles exprimées en diamètre moyen en volume de particules de charge étant mesurées par diffusion de la lumière statique au moyen d'un analyseur de taille de particules commercialisé.

**2.** Composition selon la revendication 1, dans laquelle la ou les charge(s) ayant une capacité d'absorption d'huile, mesurée au point de mouillage, supérieure ou égal à 70 ml/100 g et une taille exprimée en diamètre moyen en volume supérieure ou égale à 15 microns est (sont) présente (s) à une teneur totale supérieure ou égale à 5 % en poids, par rapport au poids total de la composition, de préférence dans la plage de 10 % à 50 % en poids, en particulier de 20 % à 40 % en poids, et plus préférablement de 25 % à 35 % en poids, par rapport au poids total de la composition.

**3.** Composition selon la revendication 1 ou 2, dans laquelle la ou les charge(s) ayant une capacité d'absorption d'huile, mesurée au point de mouillage, supérieure ou égal à 70 ml/100 g et une taille exprimée en diamètre moyen en volume supérieure ou égale à 15 microns est (sont) présente(s) selon une teneur totale supérieure ou égale à 5 % en poids, par rapport au poids total de la phase pulvérulente, de préférence dans la plage de 15 % à 60 % en poids, en particulier de 25 % à 50 % en poids, et plus préférablement de 30 % à 45 % en poids, par rapport au poids total de la phase pulvérulente.

**4.** Composition selon l'une quelconque des revendications précédentes, dans laquelle la phase pulvérulente comprend au moins une charge supplémentaire, de préférence la ou les charge(s) supplémentaire(s) est (sont) présente(s)

selon une teneur dans la plage de 15 % à 60 % en poids, par rapport au poids total de la phase pulvérulente, de préférence dans la plage de 25 % à 50 % en poids, en particulier de 30 % à 45 % en poids, par rapport au poids total de la phase pulvérulente, de préférence la ou les charge(s) supplémentaire(s) est (sont) présente(s) à une teneur totale supérieure ou égale à 5 % en poids, par rapport au poids total de la composition, de préférence dans la plage de 10 % à 50 % en poids, en particulier de 20 % à 40 % en poids, par rapport au poids total de la composition.

**5.** Composition selon la revendication précédente, dans laquelle la ou les charge(s) supplémentaire(s) est (sont) choisie(s) parmi des poudres de polyamide, des poudres d'élastomère d'organopolysiloxane, des savons métalliques, des silices, des particules d'aérogel, des (poly)oxydes métalliques, des talcs, des micas, et des mélanges de ceux-ci.

**6.** Composition selon l'une quelconque des revendications précédentes, dans laquelle le(s) agent(s) de protection contre les UV organique(s) lipophile(s) est (sont) présent(s) à une teneur totale supérieure ou égale à 0,5 % en poids, par rapport au poids total de la composition, en particulier dans la plage de 1 % à 25 % en poids par rapport au poids total de la composition, de préférence dans la plage de 4 % à 20 % en poids, encore plus préférablement de 7 % à 20 % en poids, par rapport au poids total de la composition.

**7.** Composition selon l'une quelconque des revendications précédentes, dans laquelle le(s) agent(s) de protection contre les UV organique(s) lipophile(s) est (sont) choisi(s) parmi des dérivés cinnamiques ; des anthranilates ; un dérivé salicylique, des dérivés de dibenzoylméthane, des dérivés de camphre ; des dérivés de benzophénone ; des dérivés d'acrylate de $\beta,\beta$-diphényle ; des dérivés de triazine ; des dérivés de benzotriazole ; des dérivés de phénylbenzotriazole ; des dérivés de benzalmalonate ; des imidazolines ; des dérivés d'acide p-aminobenzoïque ; des dérivés de benzoxazole ; des polymères de protection et des silicones de protection ; des dimères de dérivé de $\alpha$-alkylstyrène ; des 4,4-diarylbutadiènes ; des dérivés de mérocyanine et des mélanges de ceux-ci, de préférence comprenant au moins un dérivé cinnamique, tel que le méthoxycinnamate d'éthylhexyle, au moins un dérivé de triazine, tel que le bis-éthylhexyloxyphénol la méthoxyphényltriazine ou l'éthylhexyltriazone, au moins un dérivé de phénylbenzotriazole, tel que le drométrizole trisiloxane.

**8.** Composition selon l'une quelconque des revendications précédentes, dans laquelle le(s) agent(s) de protection contre les UV organique(s) lipophile(s) est (sont) choisi(s) parmi les butylméthoxydibenzoylméthane, méthoxycinnamate d'éthylhexyle, salicylate d'éthylhexyle, l'homosalate, butylméthoxydibenzoylméthane, l'octocrylène, 3-benzophénone, 2-(4-dléthylamino-2-hydroxybenzoyl)benzoate de n-hexyle, 4-méthylbenzylidènecamphre, bis-éthylhexyloxyphénol méthoxyphényltriazine, éthylhexyltriazone, diéthylhexylbutamidotriazone, 2,4,6-tris(4'-aminobenzalmalonate de dinéopentyle)-s-triazlne, 2,4,6-tris(4'-aminobenzalmalonate de diisobutyle)-s-triazine, 2,4-bis(4'-aminobenzalmalonate de dinéopentyle)-6-(4'-aminobenzoate de n-butyle)-s-triazine, 2,4,6-tris(biphényl-4-yl)-1,3,5-triazine, 2,4,6-tris(terphényl)-1,3,5-triazine, drométrizole trisiloxane, polysilicone-15, 1,1-dicarboxy-(2,2'-diméthylpropyl)-4,4-diphénylbutadiène, 2,4-bis[5-1-(diméthylpropyl)benzoxazol-2-yl-(4-phényl)imino]-6-(2-éthylhexyl)imino-1,3,5-triazine, et des mélanges de ceux-ci.

**9.** Composition selon l'une quelconque des revendications précédentes, dans laquelle la phase grasse liquide comprend au moins une huile, de préférence une huile non volatile, en particulier choisie parmi des huiles à base d'hydrocarbure, des huiles de silicone, et des mélanges de celles-ci.

**10.** Composition selon la revendication précédente, dans laquelle la teneur totale d'huile(s) est supérieure ou égale à 1 % en poids, par rapport au poids total de la composition, et, en particulier, est dans la plage de 1 % à 15 % en poids, par rapport au poids total de la composition.

**11.** Composition selon l'une quelconque des revendications précédentes, dans laquelle la phase pulvérulente comprend au moins un agent de protection contre les UV insoluble, en particulier choisi parmi des (poly)oxydes métalliques, en particulier un ou plusieurs (poly)oxyde(s) de titane, de préférence le (s) agent (s) de protection contre les UV insoluble(s) est (sont) présent(s) à une teneur totale supérieure ou égale à 2 % en poids, par rapport au poids total de la composition, en particulier dans la plage de 5 % à 30 % en poids, de préférence de 5 % à 10 % en poids, par rapport au poids total de la composition.

**12.** Composition selon l'une quelconque des revendications précédentes, dans laquelle la phase pulvérulente comprend au moins un colorant, en particulier choisi parmi des pigments, des nacres, des colorants, et des mélanges de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0839518 A
- WO 2013068236 A
- US 5002698 A
- US 5237071 A
- US 5166355 A
- GB 2303549 A
- DE 19726184
- EP 893119 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0518773 A
- US 5624663 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 04006878 A
- WO 05058269 A
- WO 06032741 A
- FR 2957249
- FR 2957250
- WO 2007068371 A
- WO 2008155059 A


### Non-patent literature cited in the description


- **VAN DE HULST, H.C.** Light Scattering by Small Particles. Wiley, 1957
- *The Journal of the American Chemical Society,* February 1938, vol. 60, 309
- International Cosmetic Ingredient Dictionary and Handbook. The Cosmetic, Toiletries and Fragrance Association, 1997, 371-386, 524-528
- Symmetrical Triazine Derivatives. IP.COM Journal. IP.COM INC West Henrietta, 20 September 2004
- *CHEMICAL ABSTRACTS,* 919803-06-8